(19)

**Europäisches Patentamt**

**European Patent Office**

**Office européen des brevets**

(11) **EP 2 992 823 B1**

(12) **EUROPEAN PATENT SPECIFICATION**

(45) Date of publication and mention of the grant of the patent:
**30.06.2021 Bulletin 2021/26**

(21) Application number: **14792050.8**

(22) Date of filing: **24.04.2014**

(51) Int Cl.:
*A61B 5/055* (2006.01)     *G06T 1/00* (2006.01)
*A61B 5/00* (2006.01)

(86) International application number:
**PCT/JP2014/061543**

(87) International publication number:
**WO 2014/178322 (06.11.2014 Gazette 2014/45)**

(54) **BRAIN ACTIVITY TRAINING DEVICE**

VORRICHTUNG ZUM TRAINIEREN DER HIRNAKTIVITÄT

DISPOSITIF D'ENTRAÎNEMENT CÉRÉBRAL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.05.2013 JP 2013096491**

(43) Date of publication of application:
**09.03.2016 Bulletin 2016/10**

(73) Proprietor: **Advanced Telecommunications Research Institute International**
**Soraku-gun**
**Kyoto 619-0288 (JP)**

(72) Inventors:
- **KAWATO, Mitsuo**
  **Soraku-gun**
  **Kyoto 619-0288 (JP)**
- **MORIMOTO, Jun**
  **Soraku-gun**
  **Kyoto 619-0288 (JP)**
- **YAHATA, Noriaki**
  **Soraku-gun**
  **Kyoto 619-0288 (JP)**
- **HASHIMOTO, Ryuichiro**
  **Soraku-gun**
  **Kyoto 619-0288 (JP)**
- **FUKUDA, Megumi**
  **Soraku-gun**
  **Kyoto 619-0288 (JP)**
- **SHIBATA, Kazuhisa**
  **Soraku-gun**
  **Kyoto 619-0288 (JP)**
- **IMAMIZU, Hiroshi**
  **Soraku-gun**
  **Kyoto 619-0288 (JP)**
- **WATANABE, Takeo**
  **Soraku-gun**
  **Kyoto 619-0288 (JP)**
- **SASAKI, Yuka**
  **Soraku-gun**
  **Kyoto 619-0288 (JP)**
- **KATO, Nobumasa**
  **Soraku-gun**
  **Kyoto 619-0288 (JP)**
- **KASAI, Kiyoto**
  **Soraku-gun**
  **Kyoto 619-0288 (JP)**

(74) Representative: **Müller Hoffmann & Partner Patentanwälte mbB**
**St.-Martin-Strasse 58**
**81541 München (DE)**

(56) References cited:
**WO-A1-2013/069517**

- **MICHELLE HAMPSON ET AL: "Real-time fMRI Biofeedback Targeting the Orbitofrontal Cortex for Contamination Anxiety", JOURNAL OF VISUALIZED EXPERIMENTS, no. 59, 20 January 2012 (2012-01-20), XP055331028, DOI: 10.3791/3535**

**(Cont. next page)**

- YAMASHITA O ET AL: "Sparse estimation automatically selects voxels relevant for the decoding of fMRI activity patterns", NEUROIMAGE, ELSEVIER, AMSTERDAM, NL, vol. 42, no. 4, 1 October 2008 (2008-10-01), pages 1414-1429, XP025609107, ISSN: 1053-8119, DOI: 10.1016/J.NEUROIMAGE.2008.05.050 [retrieved on 2008-06-06]
- SCHÖNFELDER VINZENZ H ET AL: "Sparse regularized regression identifies behaviorally-relevant stimulus features from psychophysical data", THE JOURNAL OF THE ACOUSTICAL SOCIETY OF AMERICA, AMERICAN INSTITUTE OF PHYSICS FOR THE ACOUSTICAL SOCIETY OF AMERICA, NEW YORK, NY, US, vol. 131, no. 5, 1 May 2012 (2012-05-01), pages 3953-3969, XP012160197, ISSN: 0001-4966, DOI: 10.1121/1.3701832 [retrieved on 2012-05-04]
- TAKEO WATANABE, YUKA SASAKI ET AL.: 'Perceptual learning incepted by decoded fMRI neurofeedback without stimulus presentation' LIFE SCIENCE SHINCHAKU RONBUN REVIEW 11 January 2012, XP055067256 Retrieved from the Internet: <URL:http://first.lifesciencedb.jp/archives/4093> [retrieved on 2014-05-15]
- KAZUHISA SHIBATA ET AL.: 'Perceptual Learning Incepted by Decoded fMRI Neurofeedback Without Stimulus Presentation' SCIENCE vol. 334, pages 1413 - 1415, XP055067256
- MEGUMI FUKUDA ET AL.: 'Decoded. Neurofeedback ni yoru Seishin Shikkan Chiryo no Kanosei' EXPERIMENTAL MEDICINE vol. 30, no. 13, 01 August 2012, pages 182.2162 - 187.2167, XP008181654
- HARDOON ET AL: "Unsupervised analysis of fMRI data using kernel canonical correlation", NEUROIMAGE, ELSEVIER, AMSTERDAM, NL, vol. 37, no. 4, 14 September 2007 (2007-09-14), pages 1250-1259, XP022250035, ISSN: 1053-8119, DOI: 10.1016/J.NEUROIMAGE.2007.06.017

**Description**

TECHNICAL FIELD

The present invention relates to a brain activity training apparatus and a brain activity training system, utilizing functional brain imaging.

BACKGROUND ART

(Biomarker)

When biological information is converted into a numerical value and quantified as an index for quantitatively comprehending biological changes in a living body, it is called a "biomarker."

According to FDA (United States Food and Drug Administration), a biomarker is regarded as "a characteristic that is objectively measured and evaluated as an indicator of normal biological processes, pathogenic processes or pharmacological responses to a therapeutic intervention." Biomarkers representative of state of disease, changes or degree of healing are used as surrogate markers (substitute markers) to monitor efficacy in clinical tests of new drugs. Blood sugar level and cholesterol level are representative biomarkers used as indexes of lifestyle diseases. Biomarkers include not only substances of biological origin contained in urine or blood but also electrocardiogram, blood pressure, PET images, bone density, lung function and the like. Developments in genomic analysis and proteome analysis have lead to discovery of various biomarkers related to DNA, RNA or biological protein.

Biomarkers are promising for measuring therapeutic efficacy after the onset of a disease and, in addition, as routine preventive indexes, promising for disease prevention. Further, application of biomarkers to individualized medicine for selecting effective treatment avoiding side effects is expected.

In the field of neurological/mental disorder, however, though studies directed to molecular markers and the like usable as objective indexes from a biochemical or molecular genetics viewpoint have been made, it will be justified to say that they are still under consideration.

Meanwhile, a disease determination system using NIRS (Near-InfraRed Spectroscopy), classifying mental disorders such as schizophrenia and depression based on features of hemoglobin signals measured by biological optical measurement, is reported (Non-Patent Literature 1).

(Real Time Neurofeedback)

Conventionally, as therapies for Obsessive-Compulsive Disorder (OCD) as one type of neurotic disease, for example, pharmacological and behavioral treatments have been known. The pharmacological treatment uses, for example, serotonin-selective reuptake inhibitor. As the behavioral treatment, exposure response prevention therapy, combining exposure therapy and response prevention has been known.

Meanwhile, real time neurofeedback is studied as a possible therapy for neurological/mental disorder.

Functional brain imaging, including functional Magnetic Resonance Imaging (fMRI), which visualizes hemodynamic reaction related to human brain activities using Magnetic Resonance Imaging (MRI), has been used to specify an active region of a brain corresponding to a component of brain function of interest, that is, to clarify functional localization of brain, by detecting difference between those in brain activities while responding to a sensory stimulus or performing a cognitive task, and those brain activities in a resting state or while performing a control task.

Recently, real time neurofeedback technique using functional brain imaging such as functional magnetic response imaging (fMRI) is reported (Non-Patent Literature 1). Real time neurofeedback technique has come to attract attention as a possible therapy of neurological disorder and mental disorder.

Neurofeedback is one type of bio-feedback, in which a subject receives feedback about his/her brain activities and thereby learns a method of managing brain activities.

By way of example, according to a report, activities of anterior cingulate cortex are measured by fMRI, the measurements are fed back to patients on real time basis as larger or smaller fire image, and the patients are instructed to make efforts to decrease the size of the fire, then improvement was attained both in real-time and long-term chronic pain of central origin (see Non-Patent Literature 2).

(Resting State fMRI)

Further, recent studies show that even when a subject is in the resting state, his/her brain works actively. Specifically, in the brain, there is a group of nerve cells that subside when the brain works actively and are excited vigorously in the resting state. Anatomically, these cells mainly exist on the medial surface where left and right cerebral hemispheres are connected such as medial aspect of the frontal lobe, posterior cingulate cortex, precuneus, posterior portion of parietal association area and middle temporal gyrus. The regions representing baseline brain activity in the resting state are named Default Mode Network (DMN) and these regions work in synchronization as one network (see Non-Patent Literature 3).

An example of difference between brain activities of a healthy individual and those of a patient of mental disease is observed in brain activities in the default mode network. The default mode network refers to portions of one's brain that exhibit more positive brain activities when a subject is in the resting state than when the subject is performing a goaldirected task. It has been reported that abnormality is observed in the default mode network of patients of mental disorder such as schizophrenia or Alzheimer's disease as compared with healthy individuals. By way of example, it is reported that in the brain of a schizophrenia patient, correlation of activities among posterior cingulate cortex, which belongs to the default mode network, and parietal lateral cortex, medial prefrontal cortex or cerebellar cortex, is decreased in the resting state.

At present, however, it is not necessarily clear how the default mode network as such relates to the cognitive function and how the correlations of functional connectivity among brain regions relates to the above-described neurofeedback.

On the other hand, changes in correlations between activities among a plurality of brain regions caused, for example, by difference in tasks are observed, so as to evaluate functional connectivity between these brain regions. Specifically, evaluation of functional connectivity in the resting state obtained by fMRI is referred to as resting-state functional connectivity MRI (rs-fcMRI), which is utilized for clinical studies directed to various neurological/mental disorders. The conventional rs-fcMRI, however, is for observing activities of global neural network such as the default mode network described above, and more detailed functional connectivity is not yet sufficiently considered.

(DecNef method: Decoded NeuroFeedback)

On the other hand, a new type neural feedback method referred to as decoded neurofeedback (DecNef) is reported recently (see Non-Patent Literature 4).

Human sensory and esthesic systems are ever-changing in accordance with the surrounding environment. Most of the changes occur in a certain early period of human developmental stage, or the period referred to as a "critical period." Adults, however, still keep sufficient degree of plasticity of sensory and esthesic systems to adapt to significant changes in surrounding environment. By way of example, it is reported that adults subjected to a training using specific esthesic stimulus or exposed to specific esthesic stimulus have improved performance for the training task or improved sensitivity to the esthesic stimulus, and that such results of training were maintained for a few months to a few years (see Non-Patent Literature 5). Such a change is referred to as sensory learning, and it has been confirmed that such a change occurs in every sensory organ, that is, vision, audition, olfaction, gustation, and taction.

According to DecNef, a stimulus as an object of learning is not directly applied to a subject while brain activities are detected and decoded, and only the degree of approximation to a desired brain activity is fed back to the subject to enable "sensory learning." See also

Non-patent Literature 7)

(Nuclear Magnetic Resonance Imaging)

Nuclear Magnetic Resonance Imaging will be briefly described in the following.

Conventionally, as a method of imaging cross-sections of the brain or the whole body of a living body, nuclear magnetic resonance imaging has been used, for example, for human clinical diagnostic imaging, which method utilizes nuclear magnetic resonance with atoms in the living body, particularly with atomic nuclei of hydrogen atoms.

As compared with "X-ray CT," which is a similar method of human tomographic imaging, characteristics of nuclear magnetic resonance imaging when applied to a human body, for example, are as follows:

(1) An image density distribution reflecting distribution of hydrogen atoms and their signal relaxation time (reflecting strength of atomic bonding) are obtained. Therefore, the shadings present different nature of tissues, making it easier to observe difference in tissues;

(2) The magnetic field is not absorbed by bones. Therefore, a portion surrounded by a bone or bones (for example, inside one's skull, or spinal cord) can easily be observed; and

(3) Unlike X-ray, it is not harmful to human body and, hence, it has a wide range of possible applications.

Nuclear magnetic resonance imaging described above uses magnetic property of hydrogen atomic nuclei (protons), which are most abundant in human cells and have highest magnetism. Motion in a magnetic field of spin angular momentum associated with the magnetism of hydrogen atomic nucleus is, classically, compared to precession of spin of a spinning top.

In the following, as a description of background of the present invention, the principle of magnetic resonance will be summarized using the intuitive classical model.

The direction of spin angular momentum of hydrogen atomic nucleus (direction of axis of rotation of spinning top) is random in an environment free of magnetic field. When a static magnetic field is applied, however, the momentum is aligned with the line of magnetic force.

In this state, when an oscillating magnetic field is superposed and the frequency of oscillating magnetic field is resonance frequency $f0 = \gamma B0/2\pi$ ($\gamma$: substance-specific coefficient) determined by the intensity of static magnetic field, energy moves to the side of atomic nuclei because of resonance, and the direction of magnetic vector changes (precession increases). When the oscillating magnetic field is turned off in this state, the precession gradually returns to the direction in the static magnetic field with the tilt angle returning to the previous angle. By externally detecting this process by an antenna coil, an NMR signal can be obtained.

The resonance frequency f0 mentioned above of hydrogen atom is $42.6 \times B0$ (MHz) where B0 (T) represents the intensity of the static magnetic field.

Further, in nuclear magnetic resonance imaging, using changes appearing in detected signals in accordance with changes in the blood flow, it is possible to visualize an active portion of a brain activated in response to an external stimulus. Such a nuclear magnetic resonance imaging is specifically referred to as fMRI (functional MRI).

An fMRI uses a common MRI apparatus with additional hardware and software necessary for fMRI measurement.

The change in blood flow causes change in NMR signal intensity, since oxygenated hemoglobin has magnetic property different from that of deoxygenated hemoglobin. Hemoglobin is diamagnetic when oxygenated, and it does not have any influence on relaxation time of hydrogen atoms in the surrounding water. In contrast, hemoglobin is paramagnetic when deoxygenated, and it changes surrounding magnetic field. Therefore, when the brain receives any stimulus and local blood flow increases and oxygenated hemoglobin increases, the change can be detected by the MRI signals. The stimulus to a subject may include visual stimulus, audio stimulus, or performance of a prescribed task (see, for example, Non-Patent Literature 2).

In the studies of brain functions, brain activities are measured by measuring increase in nuclear magnetic resonance signal (MRI signal) of hydrogen atoms corresponding to a phenomenon that density of deoxygenated hemoglobin in red blood cells decrease in minute vein or capillary vessel (BOLD effect).

Particularly, in studies related to human motor function, brain activities are measured by the MRI apparatus as described above while a subject or subjects are performing some physical activity.

For human subjects, non-invasive measurement of brain functions is essential. In this aspect, decoding technique enabling extraction of more detailed information from fMRI data has been developed (see, for example, Non-Patent Literature 6). Specifically, pixel-by-pixel brain activity analysis (volumetric pixel: voxel) of brain by the fMRI enables estimation of stimulus input and state of recognition from spatial patterns of brain activity. The above-described DecNef is an application of such a decoding technique to a task related to sensory learning.

Non-Patent Literature 8 describes a sparse logistic regression algorithm that selects relevant voxels from fMRI data.

Non-Patent Literature 9 reports on a method of analyzing fMRI data of 16 persons, wherein an algorithm is trained on a subset of fMRI data obtained by giving the persons a task, and thereafter the algorithms is used on further fMRI data to extract some regions in the brains of the persons related to the task.

CITATION LIST

PATENT LITERATURE

PTL 1: National Publication No. 2006-132313
PTL 2: Japanese Patent Laying-Open No. 2011-000184

NON PATENT LITERATURE

NPL 1: Nikolaus Weiskopf, "Real-time fMRI and its application to neurofeedback", NeuroImage 62 (2012) 682-692
NPL 2: deCharms RC, Maeda F, Glover GH et al, "Control over brain activation and pain learned by using real-time functional MRI", Proc Natl Acad Sci USA 102(51), 18626-18631, 2005

NPL 3: Raichle ME, Macleod AM, Snyder AZ, et al. "A default mode of brain function", Proc Natl Acad Sci USA 98(2), 676-682, 2001

NPL 4: Kazuhisa Shibata, Takeo Watanabe, Yuka Sasaki, Mitsuo Kawato, "Perceptual Learning Incepted by Decoded fMRI Neurofeedback Without Stimulus Presentation", SCIENCE VOL 334 9 DECEMBER 2011

NPL 5: T. Watanabe, J. E. Nanez Sr, S. Koyama, I. Mukai, J. Liederman and Y. Sasaki: Greater plasticity in lower-level than higher-level visual motion processing in a passive perceptual learning task. Nature Neuroscience, 5, 1003-1009, 2002.

NPL 6: Kamitani Y, Tong F. Decoding the visual and subjective contents of the human brain. Nat Neurosci. 2005; 8: 679-85.

NPL 7: Michelle Hampson et. al.: "Real-time fMRI Biofeedback Targeting the Orbitofrontal Cortex for Contamination Anxiety ", Journal of visualized experiments, no. 59, 20 January 2012

NPL 8: Yamashita, O. et. al.: "Sparse estimation automatically selects voxels relevant for the decoding of fMRI activity patterns. ", Neuroimage, Elsevier, vol. 42, no. 4, 1 October 2008, pages 1414-1429.

NPL 9: Hardoon, D. R. et al.: "Unsupervised analysis of fMRI data using kernel canonical correlation. ", Neuroimage, Elsevier, vol. 37, no. 4, 14 September 2007, pages 1250-1259.

## SUMMARY OF INVENTION

### TECHNICAL PROBLEM

As described above, it is noted that some of the brain activity analyses using functional brain imaging such as functional magnetic resonance imaging, and neurofeedback techniques using the same, are applicable to treatment of neurological/mental disorder. These methods and techniques, however, are not yet close to practical use.

When we consider application to treatment of neurological/mental disorder, brain activity analysis by functional brain imaging as the above-described biomarker is promising as non-invasive functional marker, and applications to development of diagnostic method and to searching/identification of target molecule for drug discovery for realizing basic remedy are also expected.

By way of example, consider mental disorder such as autism. Practical biomarker using genes is not yet established and, therefore, development of therapeutic agents remains difficult, since it is difficult to determine effect of medication.

Meanwhile, it has been suggested that diagnostic result of neurological disorder is predicable to some extent based on connections among brain regions derived from fMRI data of the resting state. To verify the prediction performance, however, these studies use only brain functions measured in one facility and, hence, usability as a biomarker has not yet been sufficiently verified.

It is noted, however, that if a biomarker allowing prediction of diagnostic result of neurological disorder based on the connections among brain regions derived from fMRI data can be realized and combined with the neurofeedback such as DecNef described above, it will be possible to realize a system for treating neurological/mental disorder.

Further, besides the treatment of neurological/mental disorder, it is desirable to enable autonomous training to attain more desirable brain state.

The present invention was made to solve the above-described problems, and its object is to provide a brain activity training apparatus and a brain activity training system, enabling training to change correlations of connections among brain regions, using correlations of connections among brain regions measured by functional brain imaging as feedback information.

Another object of the present invention is to provide a brain activity training apparatus and a brain activity training system, for treatment by changing correlations of connections among brain regions, using correlations of connections among brain regions measured by functional brain imaging as a biomarker.

### SOLUTION TO PROBLEM

According to an aspect, the present invention provides a brain activity training apparatus, including: a brain activity detecting device for time-sequentially detecting signals indicative of brain activities at a plurality of prescribed regions in a brain of a first subject; and a storage device for storing information that specifies a discriminator generated from signals measured in advance by time-sequentially measuring signals indicative of the brain activities at the plurality of prescribed regions in a brain of each of a plurality of second subjects different from the first subject. The discriminator executes a discrimination of a target attribute among attributes of the second subjects by contraction expression extracted, from correlations of brain activities among the plurality of prescribed regions, commonly with respect to at least attributes of the plurality of second subjects. The brain activity training apparatus further includes: a presenting device; and a processing device. The processing device is configured to i) calculate correlations of brain activities from among the plurality of prescribed regions, based on signals detected by the brain activity detecting device; ii) based on the calculated correlations, by the discriminator specified by the information stored in the storage device, calculate a reward value in accordance with degree of similarity of the calculated correlations to target correlations corresponding to the target attribute; and iii) present information indicative of magnitude of the reward value to the subject by the presenting device.

Preferably, the signals measured in advance from the plurality of second subjects are measured by a plurality of brain activity measuring devices; and the contraction expression used by the discriminator is a contraction expression extracted, by variable selection from correlations of brain activities among the plurality of prescribed regions, commonly with respect to measuring conditions of the plurality of brain activity measuring devices, and attributes of the plurality of subjects, extracted.

Preferably, the discriminator is generated by regression of performing further variable selection on the extracted contraction expression.

Preferably, the discriminator is generated by sparse logistic regression on sparse non-diagonal elements and a target attribute of the second subjects. The sparse non-diagonal elements are made sparse based on a result obtained by regularized canonical correlation analysis with respect to non-diagonal elements of correlation matrix of brain activities at the plurality of prescribed regions of the second subjects and the attributes of the second subjects. An input to the discriminator is a linear weighted sum of the non-diagonal elements of the first subject corresponding to the result of the regularized canonical correlation analysis.

Preferably, the discriminator is generated by sparse logistic regression on sparse non-diagonal elements and a target attribute of the second subjects. The sparse non-diagonal elements are made sparse based on a result obtained by regularized canonical correlation analysis with respect to non-diagonal elements of correlation matrix of brain activities at the plurality of prescribed regions of the second subjects and on the attributes of the second subjects. An input to the discriminator is a non-diagonal element selected as related to the target attribute, from the non-diagonal elements based on a result of the regularized canonical correlation analysis.

Preferably, the brain activity detecting device includes a brain activity detecting device for picking-up a resting-state functional connectivity magnetic resonance image.

Preferably, the regularized canonical correlation analysis is canonical correlation analysis with L1 regularization.

According to another aspect, the present invention provides a brain activity training system, including: a brain activity detecting device for time-sequentially detecting signals indicative of brain activities at a plurality of prescribed regions in a brain of a first subject; and discriminator generating means for generating a discriminator from signals measured in advance by the brain activity detecting device by time-sequentially measuring signals indicative of the brain activities at the plurality of prescribed regions in a brain of each of a plurality of second subjects different from the first subject. The discriminator generating means extracts contraction expression common to at least attributes of the plurality of second subjects from correlations of brain activities among the plurality of prescribed regions and generates a discriminator for the extracted contraction expression with respect to a target attribute among the attributes of the second subjects. The brain activity training system further includes: a storage device for storing information that specifies the discriminator; a presenting device; and a processing device. The processing device is configured to i) calculate correlations of brain activities from among the plurality of prescribed regions, based on signals detected by the brain activity detecting device; ii) based on the calculated correlations, by a discriminant process by the discriminator specified by the information stored in the storage device, calculate a reward value in accordance with degree of similarity of the calculated correlations to target correlations corresponding to the target attribute; and iii) present information indicative of magnitude of the reward value to the subject by the presenting device.

Preferably, the brain activity detecting device includes a plurality of brain activity measuring devices. The discriminator generating means includes extracting means for extracting, by variable selection from correlations of brain activities among the plurality of prescribed regions, a contraction expression common to measuring conditions of the plurality of brain activity measuring devices, and attributes of the plurality of subjects.

Preferably, the discriminator generating means includes regression means for generating the discriminator by regression of performing further variable selection on the extracted contraction expression.

Preferably, the extracting means includes correlation analyzing means for calculating a correlation matrix of activities at the plurality of prescribed regions from the signals detected by the brain activity detecting device, executing regularized canonical correlation analysis between attributes of the subjects and non-diagonal elements of the correlation matrix and thereby for extracting the contracted expression.

Preferably, the regression means includes regression analysis means for generating a discriminator by sparse logistic regression on a result of the regularized canonical correlation analysis and the attributes of the subjects.

The plurality of brain activity measuring devices are devices for time-sequentially measuring brain activities by functional brain imaging installed at a plurality of different locations, respectively.

The discriminant process is discrimination of a disease label indicating whether the first subject is healthy or a patient of a neurological/mental disorder.

The attributes of the subjects include a disease label indicating whether the subject is healthy or a patient of a neurological/mental disorder, a label indicating individual nature of the subject, and information characterizing measurement by the brain activity detecting device. The discriminant process is discrimination of a disease label indicating whether the first subject is healthy or a patient of a neurological/mental disorder.

The regularized canonical correlation analysis is canonical correlation analysis with L1 regularization.

The brain activity measuring device picks up a resting-state functional connectivity magnetic resonance image.

ADVANTAGEOUS EFFECTS OF INVENTION

According to the present invention, correlations of connections among brain regions measured by functional brain imaging are used as feedback information, which will enable a subject to change the correlations of connections among brain regions by training.

BRIEF DESCRIPTION OF DRAWINGS

Fig. 1 is a schematic diagram showing an overall configuration of an MRI apparatus 10.

Fig. 2 is a hardware block diagram of data processing unit 32.

Fig. 3 shows regions of interest (ROI) of a brain imaged by rs-fcMRI in accordance with an embodiment.

Fig. 4 shows a concept of a procedure for extracting correlation matrix representing correlations of functional connectivity in the resting state.

Fig. 5 shows a concept of a process for generating a discriminator serving as a biomarker, from the correlation matrix.

Fig. 6 is a flowchart representing a process executed by data processing unit 32 for generating the discriminator serving as the biomarker.

Fig. 7 shows features obtained by Sparse Canonical Correlation Analysis (SCCA) (SCCA feature space) and a concept of a procedure for generating the discriminator by and SLR using the features as an input.

Fig. 8 shows features (SCCA feature space) obtained by SCCA and a concept of a procedure for generating the discriminator by SLR using the features as an input.

Fig. 9 shows features (SCCA feature space) obtained by SCCA and a concept of a procedure for generating the discriminator using by SLR using the features as an input.

Fig. 10 shows a concept of generating a biomarker.

Fig. 11 shows a concept of verifying the generated biomarker.

Fig. 12 shows properties of the biomarker.

Fig. 13 shows a conceptual configuration of brain activity training apparatus.

Fig. 14 is a flowchart representing a process executed by data processing unit 32 for decoded connectivity neurofeedback.

Fig. 15 is a flowchart representing a process for deriving a calibration bias term.

Fig. 16 is a flowchart representing a score calculating process for measurements in resting states before and after training.

Fig. 17 shows an example of a training sequence in the decoded connectivity neurofeedback.

Fig. 18 shows in-brain positions of lateral parietal area LP and primary motor cortex M1.

Fig. 19 shows a training sequence of the decoded connectivity neurofeedback.

Fig. 20 shows changes between initial stage and terminal stage of brain function correlations (connectivity) training of a subject of a first group.

Fig. 21 shows results of measurement of brain activity in the resting state after the last training.

Fig. 22 shows brain activity correlations by the measurement of brain activity in the resting state before training and brain activity correlations by the measurement of brain activity in the resting state after training.

Fig. 23 shows changes in scores when decoded connectivity neurofeedback is performed on a subject diagnosed as having autism.

DESCRIPTION OF EMBODIMENTS

In the following, a configuration of an MRI system in accordance with embodiments of the present invention will be described with reference to the drawings. In the embodiments below, components or process steps denoted by the same reference characters are the same or corresponding components or steps and, therefore, description thereof will not be repeated unless necessary.

[First Embodiment]

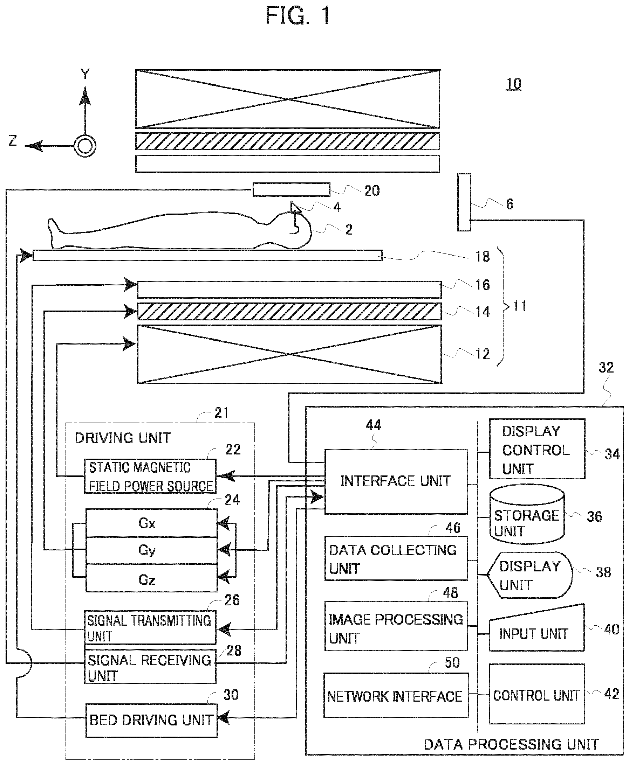

Fig. 1 is a schematic diagram showing an overall configuration of an MRI apparatus 10.

Referring to Fig. 1, MRI apparatus 10 includes: a magnetic field applying mechanism 11 applying a controlled magnetic field to, and irradiating with RF wave, a region of interest of a subject 2; a receiving coil 20 receiving a response wave (NMR signal) from subject 2 and outputting an analog signal; a driving unit 21 controlling the magnetic field applied to subject 2 and controlling transmission/reception of RF wave; and a data processing unit 32 configuring a control sequence of driving unit 21 and processing various data signals to generate an image.

Here, a central axis of a cylindrical bore in which subject 2 is placed is regarded as a Z-axis, and a horizontal direction orthogonal to the Z-axis and the vertical direction orthogonal to the Z-axis are defined as X-axis and Y-axis, respectively.

In MRI apparatus 10 having such a configuration, because of the static magnetic field applied by magnetic field applying mechanism 11, nuclear spins of atomic nuclei forming subject 2 are oriented in the direction of magnetic field (Z-axis) and perform precession with the direction of magnetic field being an axis, with Larmor frequency unique to the atomic nuclei.

When irradiated with an RF pulse of the same Larmor frequency, the atoms resonate, absorb energy and are excited, resulting in nuclear magnetic resonance (NMR). When the irradiation with RF pulse is stopped after the resonance, the atoms discharge energy and return to the original, steady state. This process is referred to as a relaxation process. In the relaxation process, the atoms output electromagnetic wave (NMR signal) having the same frequency as the Larmor frequency.

The output NMR signal is received by receiving coil 20 as a response wave from subject 2, and the region of interest of subject 2 is imaged by data processing unit 32.

Magnetic field applying mechanism 11 includes a static magnetic field generating coil 12, a magnetic field gradient generating coil 14, an RF irradiating unit 16, and a bed 18 for placing subject 2 in the bore.

By way of example, subject 2 lies on his/her back on bed 18. Though not limited, subject 2 may view an image displayed on a display 6 mounted vertical to the Z-axis, using prism glasses 4. Visual stimulus is applied to subject 2 by an image on display 6. In another embodiment, visual stimulus to subject 2 may be applied by projecting an image in front of subject 2 using a projector.

Such a visual stimulus corresponds to presentation of feedback information in the above-described neurofeedback.

Driving unit 21 includes a static magnetic field power source 22, a magnetic field gradient power source 24, a signal transmitting unit 26, a signal receiving unit 28, and a bed driving unit 30 for moving bed 18 to any position along the Z-axis.

Data processing unit 32 includes: an input unit 40 for receiving various operations and information input from an operator (not shown); a display unit 38 for displaying various images and various pieces of information related to the region of interest of subject 2, on a screen; a display control unit 34 for controlling display of display unit 38; a storage unit 36 for storing programs to cause execution of various processes, control parameters, image data (structural images and the like) and other electronic data; a control unit 42 controlling operations of various functional units, including generating a control sequence for driving the driving unit 21; an interface unit 44 for executing transmission/reception of various signals to/from driving unit 21; a data collecting unit 46 for collecting data consisting of a group of NMR signals derived from the regions of interest; an image processing unit 48 for forming an image based on the data of NMR signals; and a network interface 50 for executing communication with a network.

Data processing unit 32 may be a dedicated computer, or it may be a general purpose computer executing functions of causing operations of various functional units, in which designated operations, data processing and generation of control sequence are realized by a program or programs stored in storage unit 36. In the following, description will be given assuming that data processing unit 32 is implemented by a general purpose computer.

Static magnetic field generating coil 12 causes a current supplied from a static magnetic field power source 22 to flow through a helical coil wound around the Z-axis to generate an induction magnetic field, and thereby generates a static magnetic field in the Z-direction in the bore. The region of interest of subject 2 is placed in the region of highly uniform static magnetic field formed in the bore. More specifically, here, static magnetic field generating coil 12 is comprised of four air core coils, forms a uniform magnetic field inside by the combination of the coils, and attains orientation of the spins of prescribed atomic nuclei in the body of subject 2, or more specifically, the spins of hydrogen atomic nuclei.

Magnetic field gradient generating coil 14 is formed of X-, Y- and Z-coils (not shown), and provided on an inner peripheral surface of cylindrical static magnetic field generating coil 12.

These X-, Y- and Z- coils superpose magnetic field gradients on the uniform magnetic field in the bore with the X-axis, Y-axis and Z-axis directions switched in turn, whereby creating intensity gradient in the static magnetic field. When excited, the Z-coil tilts the magnetic field intensity to the Z-direction and thereby defines a resonance surface; the Y-coil applies a tilt for a short period of time immediately after application of the magnetic field in the Z-direction, and thereby adds phase modulation in proportion to the Y-coordinate, to the detected signal (phase encoding); and thereafter the X-coil applies a tilt when data is collected, and thereby adds frequency modulation in proportion to the X-coordinate, to the detected signal (frequency encoding).

The switching of superposed magnetic field gradients is realized as different pulse signals are output to the X-, Y- and Z-coils from the magnetic field gradient power source 24 in accordance with a control sequence. Thus, the position of subject 2 expressed by the NMR can be specified, and positional information in three-dimensional coordinates necessary for forming an image of subject 2 are provided.

Here, using the orthogonally crossing three sets of magnetic field gradients, allocating slice direction, phase encoding direction and frequency encoding direction to the magnetic fields respectively and by combining these, images can be taken from various angles. By way of example, in addition to transverse slice in the same direction as taken by an X-ray CT apparatus, saggital and coronal slices orthogonal thereto, as well as an oblique slice, of which direction vertical to its plane is not parallel to any of the axes of three orthogonally crossing magnetic field gradients, can be imaged.

RF irradiating unit 16 irradiates a region of interest of subject 2 with RF (Radio Frequency) pulses based on a high-frequency signal transmitted from a signal transmitting unit 26 in accordance with a control sequence.

Though RF irradiating unit 16 is built in magnetic field applying mechanism 11 in Fig. 1, it may be mounted on bed 18 or integrated with receiving coil 20.

Receiving coil 20 detects a response wave (NMR signal) from subject 2, and in order to detect the NMR signal with high sensitivity, it is arranged close to subject 2.

Here, when an electromagnetic wave of NMR signal crosses a coil strand of receiving coil 20, a weak current is generated by electromagnetic induction. The weak current is amplified by signal receiving unit 28 and converted from an analog signal to a digital signal, and then transmitted to data processing unit 32.

The mechanism here is as follows. To a subject 2 in a state of static magnetic field with Z-axis magnetic field gradient added, a high-frequency electromagnetic field of resonance frequency is applied through RF irradiating unit 16. Prescribed atomic nuclei at a portion where magnetic field intensity satisfies the condition of resonance, for example, hydrogen atomic nuclei, are selectively excited and start resonating. Prescribed atomic nuclei at a portion satisfying the condition of resonance (for example, a slice of prescribed thickness of subject 2) are excited, and spin axes of atomic nuclei concurrently start precession. When the excitation pulse is stopped, electromagnetic waves irradiated by the atomic nuclei in precession induce a signal in receiving coil 20 and, for some time, this signal is continuously detected. By this signal, a tissue containing the prescribed atoms in the body of subject 2 is monitored. In order to know the position where the signal comes from, X- and Y-magnetic field gradients are added and the signal is detected.

Based on the data built in storage unit 36, image processing unit 48 measures detected signals while repeatedly applying excitation signals, reduces resonance frequency to X-coordinate by a first Fourier transform, restores Y-coordinate by a second Fourier transform, and thus, displays a corresponding image on display unit 38.

For example, by picking-up the above-described BOLD signal on real-time basis using the MRI system as described above and performing an analysis, which will be described later, on the time-sequentially picked-up images by control unit 42, it is possible to take resting-state functional connectivity MRI (rs-fcMRI).

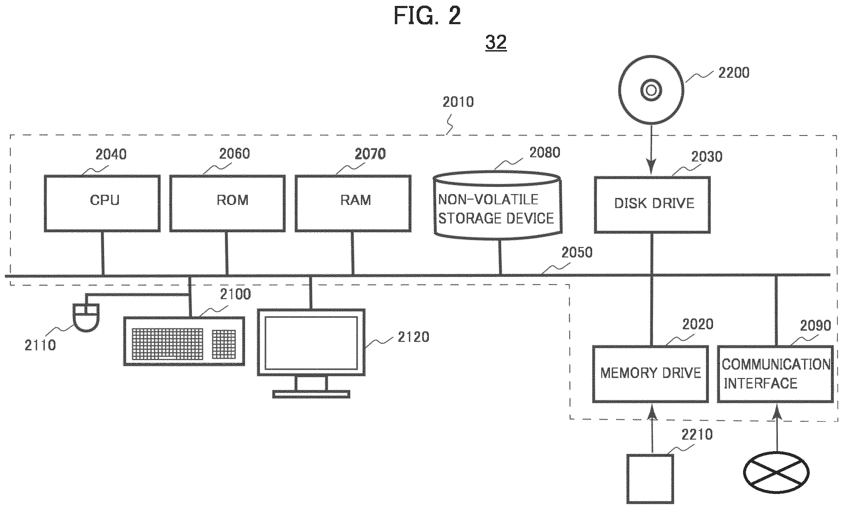

Fig. 2 is a hardware block diagram of data processing unit 32.

Though the hardware of data processing unit 32 is not specifically limited as described above, a general-purpose computer may be used.

Referring to Fig. 2, a computer main body 2010 of data processing unit 32 includes, in addition to a memory drive 2020 and a disk drive 2030, a CPU 2040, a bus 2050 connected to disk drive 2030 and memory drive 2020, an ROM 2060 for storing programs such as a bootup program, an RAM 2070 for temporarily storing instructions of an application program and providing a temporary memory space, a non-volatile storage device 2080 for storing an application program, a system program and data, and a communication interface 2090. Communication interface 2090 corresponds to an interface unit 44 for transmitting/receiving signals to/from driving unit 21 and the like and a network interface 50 for communicating with another computer through a network, not shown. As non-volatile storage device 2080, a hard disk (HDD), a solid state drive (SSD) or the like may be used.

By operation processes executed by CPU 2040 in accordance with a program, various functions of data processing unit 32 including functions of control unit 42, data collecting unit 46 and image processing unit 48 are realized.

A program or programs causing data processing unit 32 to execute the function of the present embodiment as described above may be stored in a CD-ROM 2200 or a memory medium 2210 and inserted to disk drive 2030 or memory drive 2020 and may further by transferred to non-volatile storage device 2080. The program is loaded to RAM 2070 before execution.

Data processing unit 32 further includes a keyboard 2100 and a mouse 2110 as input devices, and a display 2120 as an output device. Keyboard 2100 and mouse 2110 correspond to input unit 40 and display 2120 corresponds to display unit 38.

The program realizing the function of data processing unit 32 as described above may not necessarily include an operating system (OS) for executing the function of information processing apparatus such as computer main body 2010. The program may only include those portions of instructions which can call appropriate functions (modules) in a controlled manner to attain a desired result. The manner how data processing unit 32 operates is well known and, therefore, detailed description will not be given here.

It is noted that one or a plurality of computers may be used to execute the program described above. In other words, either centralized or distributed processing may be possible.

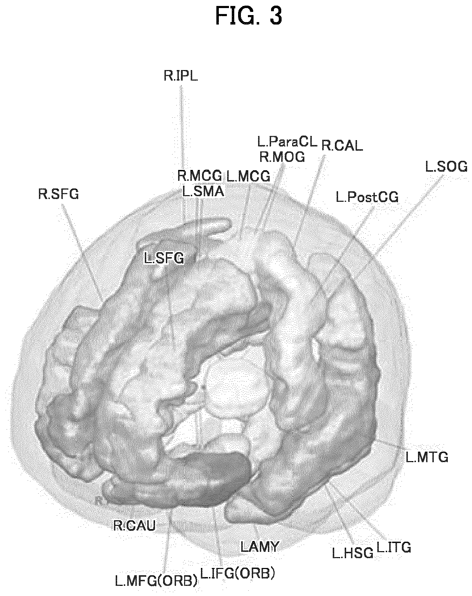

Fig. 3 shows regions of interest (ROI) of a brain imaged by rs-fcMRI in accordance with an embodiment.

Here, a biomarker related to Autistic Spectrum Disorder (ASD) will be described as an example, and ninety-three regions are used as regions of interest.

Such regions of interest include, by way of example, the following:

Dorsomedial Prefrontal Cortex (DMPFC);
Ventromedial Prefrontal Cortex (VMPFC);
Anterior Cingulate Cortex (ACC);
Cerebellar Vermis;
Left Thalamus;
Right Inferior Parietal Lobe;
Right Caudate Nucleus;
Right Middle Occipital Lobe; and
Right Middle Cingulate Cortex.

It is noted, however, that the brain regions used may not be limited to those above.

For instance, the regions to be selected may be changed in accordance with the neurological/mental disorder to be studied.

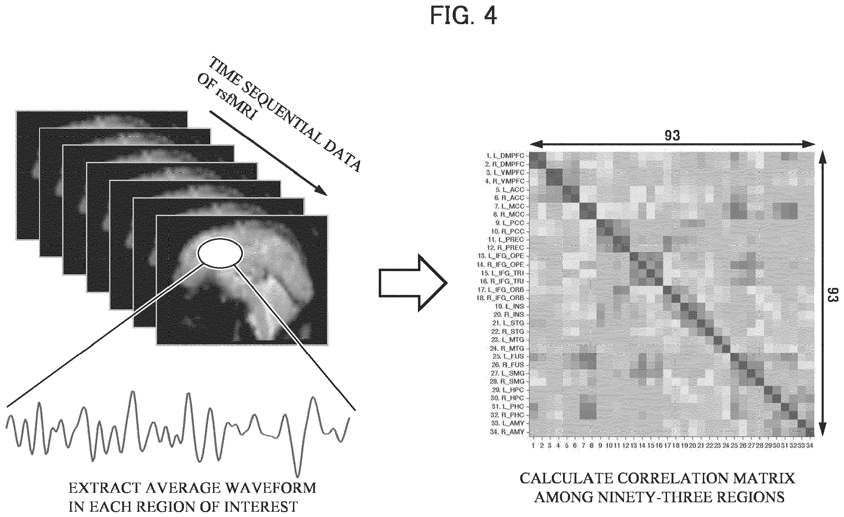

Fig. 4 shows a concept of a procedure for extracting correlation matrix representing correlations of functional connectivity in the resting state, from the regions of interest such as shown in Fig. 3.

Referring to Fig. 4, from fMRI data of n (n: natural number) time points in the resting state measured on real-time basis, average "degree of activity" of each region of interest is calculated, and correlations among the brain regions (among the regions of interest) are calculated.

Here, ninety-three regions are picked up as regions of interest and, therefore, the number of independent non-diagonal elements in the correlation matrix will be, considering the symmetry,

$$(93 \times 93 - 93)/2 = 4278.$$

In Fig. 4, only the correlations of 34 × 34 are shown.

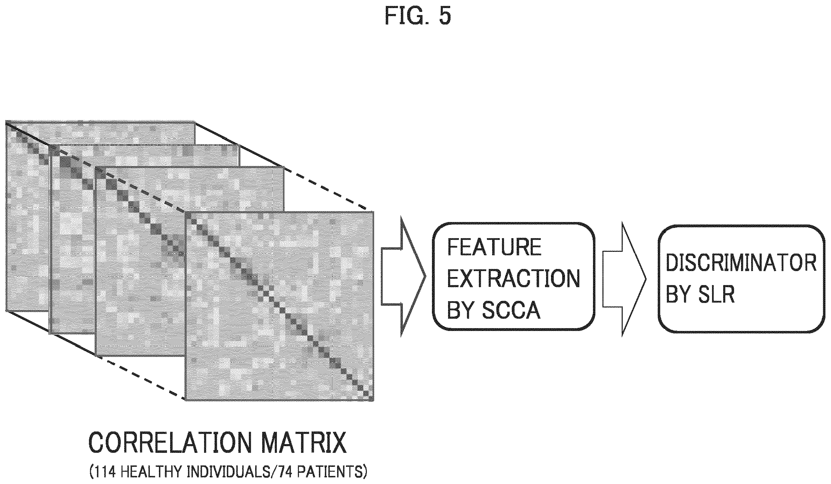

Fig. 5 shows a concept of a process for generating a discriminator serving as a biomarker, from the correlation matrix described with reference to Fig. 4.

Referring to Fig. 5, from data of resting-state functional connectivity MRI obtained by measuring a group of healthy subjects (in this example, 114 individuals) and a group of patients (in this example, seventy-four individuals), data processing unit 32 derives correlation matrix of degree of activity among brain regions (regions of interest) in accordance with a procedure that will be described later.

Thereafter, by data processing unit 32, feature extraction is performed by regularized canonical correlation analysis on the correlation matrix and on the attributes of subjects including disease/healthy labels of the subjects. Here, "regularization" generally refers to a method of preventing over-learning by adding a regularization term to an error function in machine learning and statistics and thereby restricting complexity/degree of freedom of a model. If the result of regularized canonical correlation analysis results in sparse explanatory variables this process will be specifically referred to as sparse canonical correlation analysis (SCCA). In the following, an example employing SCCA will be described.

Further, by data processing unit 32, based on the result of regularized canonical correlation analysis, a discriminator is generated from discriminant analysis by sparse logistic regression.

As will be described later, the data of healthy group and the patient group are not limited to those measured by the MRI itself. Data measured by a different MRI apparatus may also be integrated, to generate the discriminator. Generally speaking, data processing unit 32 may not necessarily be a computer for executing control of the MRI apparatus, and it may be a computer specialized in generating the discriminator by receiving measurements data from a plurality of MRI apparatuses and performing the discriminant process by using the generated discriminator.

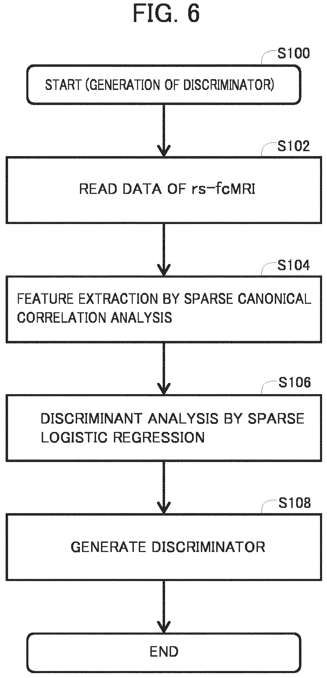

Fig. 6 is a flowchart representing a process executed by data processing unit 32 for generating the discriminator serving as the biomarker.

In the following, the process described with reference to Fig. 5 will be discussed in greater detail with reference to Fig. 6.

The biggest problem posed when a biomarker is to be generated based on the discriminant label of a disease of a subject and connections of brain regions derived from the fMRI data in the resting state is that the number of data dimensions is overwhelmingly larger than the number of data. Therefore, if training of a discriminator for predicting the disease discriminant label (here, the label indicating whether the subject has the disease or healthy will be referred to as the "disease discriminant label") is done using a data set without regularization, the discriminator will be over-fitted, and the prediction performance for unknown data significantly decreases.

Generally, in machine learning, a process to enable explanation of observed data with a smaller number of explanatory variables is referred to as "variable selection (or feature extraction)." In the present embodiment, "extraction of contraction expression" refers to variable selection (feature extraction) to enable formation of the discriminator with smaller number of correlation values in the machine learning of the discriminator for predicting the discriminant label of a disease to be studied from among "a plurality of correlation values (a plurality of connections) of the degree of activity among brain regions (regions of interest)," that is, to select correlation values of higher importance as the explanatory variables.

In the present embodiment, as the method of feature extraction, regularization is adopted. In this manner, canonical correlation analysis is performed with regularization and obtaining sparse variables, so as to leave explanatory variables of higher importance. This process is referred to as sparse canonical correlation analysis. More specifically, as the method of regularization also results in sparse variables, we can use a method of imposing a penalty to the magnitude of absolute value of parameters for canonical correlation analysis referred to as "L1 regularization" as will be described in the following.

Specifically, referring to Fig. 6, when the process for generating the discriminator starts (S100), data processing unit 32 reads rs-fcMRI data of each subject from storage unit 36 (S102), and performs feature extraction by SCCA (S104).

In the following, L1 regularization canonical correlation analysis will be described. As to the L1 regularization canonical correlation analysis, see, for example, the reference below.

Reference: Witten DM, Tibshirani R, and T Hastie. A penalized matrix decomposition, with applications to sparse principal components and canonical correlation analysis. Biostatistics, Vol. 10, No. 3, pp. 515-534, 2009.

First, in general Canonical Correlation Analysis (CCA), a data pair $x_1$ and $x_2$ as given below is considered. It is noted that the variables $x_1$ and $x_2$ are standardized to have an average zero and standard deviation one. Further, it is assumed that each of the data pair $x_1$ and $x_2$ has the data number of n.

$\mathbf{x}_1 \in R^{n \times p_1}$, $\mathbf{x}_2 \in R^{n \times p_2}$

Here, according to CCA, parameters $\mathbf{w}_1 \in R^{p_1}$, $\mathbf{w}_2 \in R^{p_2}$ that can maximize the correlation between $\mathbf{z_1} = \mathbf{x}_1\mathbf{w}_1$, $\mathbf{z_2} = \mathbf{x}_2\mathbf{w}_2$ are calculated. That is, the following optimization problem is solved. Under the condition of

$$\mathbf{w}_1^T \mathbf{x}_1^T \mathbf{x}_1 \mathbf{w}_1 = \mathbf{w}_2^T \mathbf{x}_2^T \mathbf{x}_2 \mathbf{w}_2 = 1$$
$$\max_{\mathbf{w}_1, \mathbf{w}_2} \mathbf{w}_1^T \mathbf{x}_1^T \mathbf{x}_2 \mathbf{w}_2 .$$

In contrast, by introducing L1 regularization, the process will be to solve the following optimization problem.

Under the condition of

$$\left\| \mathbf{w}_1 \right\|^2 \le 1, \quad \left\| \mathbf{w}_2 \right\|^2 \le 1, \quad \left\| \mathbf{w}_1 \right\|_1 \le c_1, \quad \left\| \mathbf{w}_2 \right\|_1 \le c_2,$$
$$\max_{\mathbf{w}_1, \mathbf{w}_2} \mathbf{w}_1^T \mathbf{x}_1^T \mathbf{x}_2 \mathbf{w}_2$$

Here, $c_1$ and $c_2$ are parameters representing the strength of L1 regularization, and they are set appropriately in accordance with the data by various known methods. The suffix "1" on the lower right side of $\|w_1\|_1$ and $\|w_2\|_1$ represents that $\|w_1\|$ and $\|w_2\|$ are L1 norms.

As the constraint condition for L1 regularization is added, the values of those elements of lower importance of the elements of parameters $w_1$ and $w_2$ will become zero and, hence, the features (explanatory variables) become sparse.

Thereafter, data processing unit 32 performs discriminant analysis by SLR based on the result of SCCA (S106).

SLR refers to a method of logistic regression analysis expanded to a frame of Bayes' estimation, in which dimensional compression of a feature vector is performed concurrently with weight estimation for discriminant. This is useful when the feature vector of data has a very large number of dimensions and includes many unnecessary feature elements. For unnecessary feature elements, weight parameter in linear discriminant analysis will be set to zero (that is, variable selection is done), so that only a limited number of feature elements related to the discriminant are extracted (sparseness).

In SLR, probability p of obtained feature data belonging to a class is calculated class by class, and the feature data is classified to the class corresponding to the highest output value p. The value p is output by a logistic regression equation. Weight estimation is done by ARD (Automatic Relevance Determination), and feature element less contributing to class determination is removed from the calculation as its weight comes closer to zero.

Specifically, using the features extracted by the L1 regularization CCA as described above, the discriminator based on the hierarchical Bayes' estimation as will be described in the following estimates the disease/healthy label.

Here, assuming that the features $z_1 = w_1 x_1$ derived from CCA are input to SLR and S = {0, 1} is a label of disease (S = 1)/healthy (S = 0), then, the probability of the SLR output being S = 1 is defined by Equation (1) below.

$$p(\mathbf{z}_1, \mathbf{w}) = \frac{1}{1 + e^{-\mathbf{w}^T \mathbf{z}_1}} \quad \cdots (1)$$

Here, the distribution of parameter vector w is set to the normal distribution as given below. In the equation, $\alpha$ represents a hyper parameter vector representing variance of normal distribution of vector w.

$$p(\mathbf{w} \mid \alpha) = N(\mathbf{w} \mid 0, diag(\alpha))$$

Further, by setting the distribution of hyper parameter vector $\alpha$ as follows, distribution of each parameter is estimated by hierarchical Bayes' estimation.

$$p(\alpha) = \prod_j \Gamma(\alpha_i \mid a^0, b^0)$$

Here, $\Gamma$ represents a $\Gamma$ distribution, and $a^0$ and $b^0$ are parameters determining gamma distribution of hyper parameter vector $\alpha$. The i-th element of vector $\alpha$ is represented by $\alpha_i$.

As to the SLR, see, for example, the reference below.

Reference: Okito Yamashita, Masaaki Sato, Taku Yoshioka, Frank Tong, and Yukiyasu Kamitani. "Sparse Estimation automatically selects voxels relevant for the decoding of fMRI activity patterns." NeuroImage, Vol. 42, No. 4, pp. 1414-1429, 2008.

Based on the result of discriminant analysis as described above, the discriminator that discriminates the disease/healthy label (label for discriminating the disease) on the input features is generated (S108). The information for specifying the generated discriminator (data related to the function form and parameters) is stored in storage unit 36, and, when test data is input later, will be used for the discriminant process when the discriminant label of the disease is estimated for the test data.

Specifically, based on doctors' diagnosis in advance, the subjects are divided to a group of healthy individuals and a group of patients. Correlations (connectivity) of degree of activities among brain regions (regions of interest) of the subjects will be measured. By machine learning of measurement results, the discriminator is generated to discriminate whether test data of a new subject, different from those above, fits to disease or healthy. The discriminator functions as a biomarker of mental disorder. Here, the "disease discriminant label" as the biomarker output may include a probability that the subject has the disease (or probability that the subject is healthy), since the discriminator is generated by logistic regression. Such a probability may be used as a "diagnosis marker."

That is to say, the discriminator generated in this manner functions as a biomarker for a mental disorder.

In summary, in biomarker learning (generation), in order to generate the biomarker for mental disorder, data of resting state functional connectivity MRI (rs-fcMRI) is used as an input, feature extraction is done by L1 regularization CA as described above, and using the extracted features as the input, disease/healthy discriminant is done by the SLR.

In the foregoing, it is assumed that the features $z_1 = w_1 x_1$ derived from CCA are used as the features to be the input to SLR.

The features to be used as the input to SLR, however, are not limited to the above.

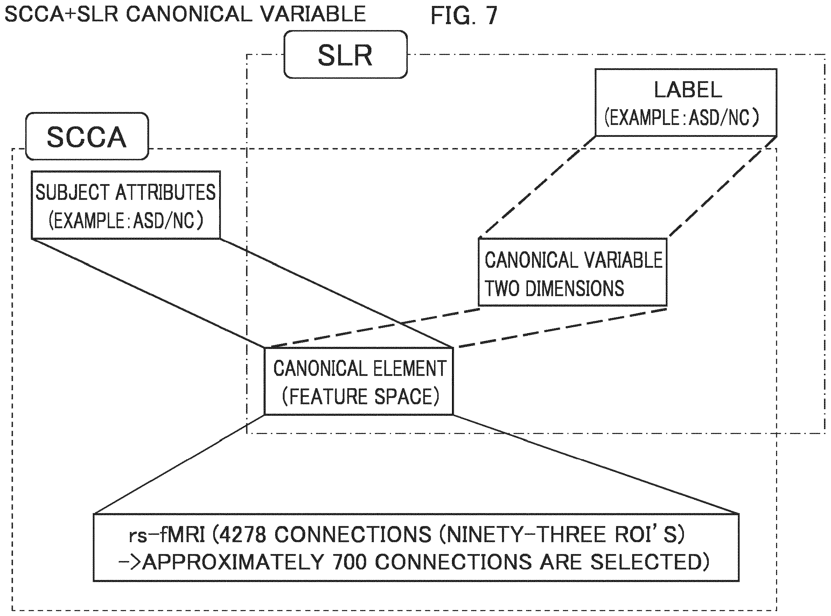

Figs. 7 to 9 show a concept of a procedure for generating the discriminator using features obtained by SCCA (SCCA feature space) and SLR using the features as an input.

First, as shown in Fig. 7, as a first method, using healthy/disease label as an attribute of subjects, SCCA is executed between the label and the elements of correlation matrix obtained from rs-fcMRI.

The resulting features (intermediate expression) $z_1 = w_1 x_1$ (canonical variance, two dimensions) are used as an input to SLR, and the discriminator is generated (hereinafter this procedure will be referred to as "method A").

Here, "canonical variance, two dimensions" means that two dimensional vectors of disease (1, 0) and healthy (0, 1) are used.

Here, of 4278 of non-diagonal elements of correlation matrix, approximately 700 elements (connections) are selected by SCCA.

Specifically, using certain data of disease/healthy diagnosis results, parameters of discriminator are determined in advance by machine learning such as SCCA, SLR or the like. The determined parameters are generalized for unknown rs-fcMRI data, and thus, the discriminator functions as a biomarker. For the discriminator, linear sum of selected connections of rs-fcMRI is applied as an input, and the disease label (1, 0) and the healthy label (0, 1) correspond to the output.

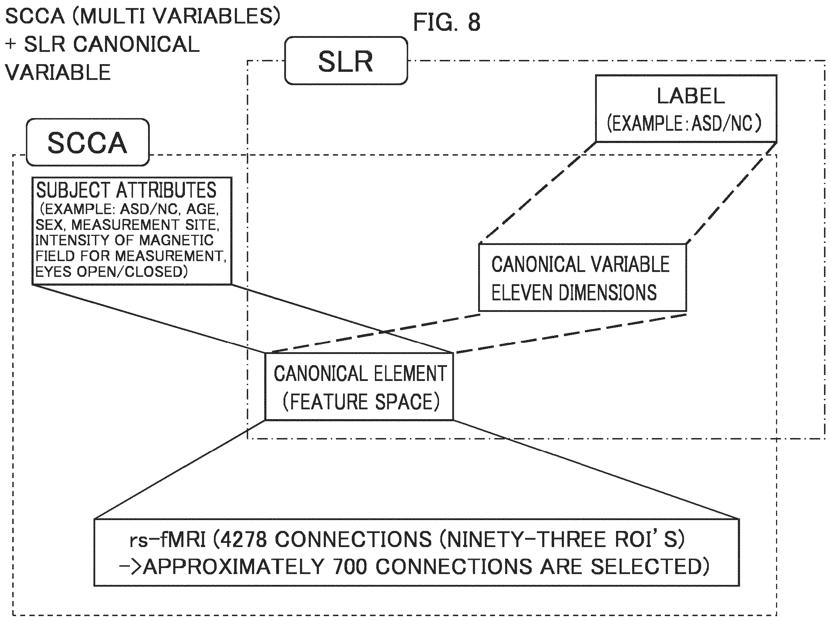

Referring to Fig. 8, as the second method, the following procedure may be adopted. Attributes of subjects include the disease/healthy label, age of the subject, sex of the subject, measurement agency (the place where the measuring apparatus is placed, referred to as measurement site; in the example described later, the number of measurement sites is three), intensity of magnetic field applied by the fMRI apparatus (corresponding to one of the indexes of performance such as the resolution of imaging apparatus), and conditions of experiments such as eyes opened/closed at the time of imaging are used. SCCA is executed between these attributes and the elements of correlation matrix obtained from rs-fcMRI. As the index of performance of fMRI imaging apparatus, not only the intensity of applied magnetic field but also other indexes may be used.

In other words, as the "explanatory variables" of canonical correlation analysis, independent elements of correlation matrix from rs-fcMRI are adopted, and as the "criterion variables," the attributes of subject such as described above are adopted.

The resulting features (intermediate expression) $z_1 = w_1 x_1$ (canonical variance, eleven dimensions) are used as an input to SLR, and the discriminator is generated (hereinafter this procedure will be referred to as "method B").

Here, "canonical variance, eleven dimensions" means a sum of eleven dimensions including disease/healthy (two dimensions), three sites (three dimensions: (1, 0, 0), (0, 1, 0), (0, 0, 1)), age (one dimension), sex (two dimensions (1, 0), (0, 1)), performance (one dimension), eyes opened/closed (two dimensions: (1, 0), (0, 1)).

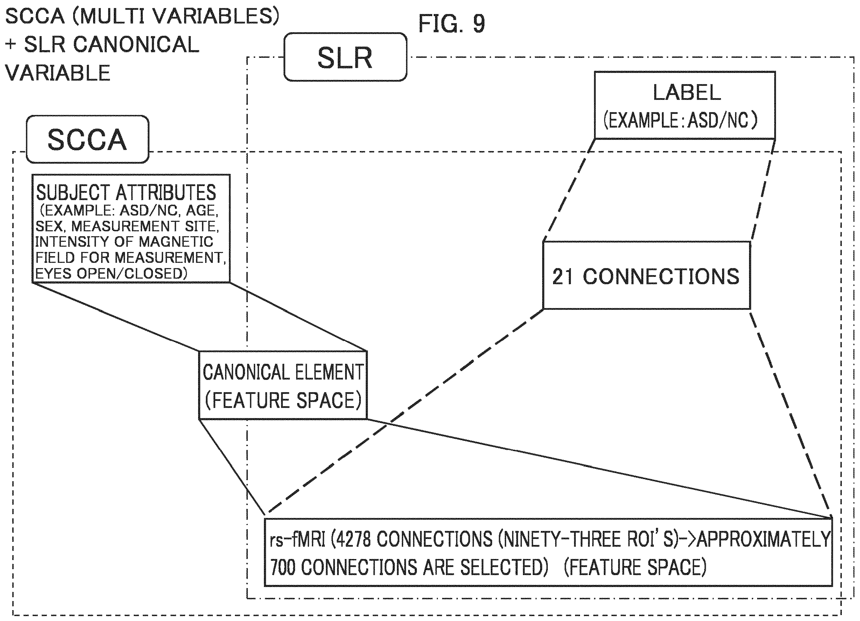

Fig. 9 shows a third method. In the third method, as attributes of subjects, the disease/healthy label, age of the subject, sex of the subject, measurement agency (measurement site), performance of fMRI apparatus (example: intensity of magnetic field applied), and conditions of experiments such as eyes opened/closed at the time of imaging are used. SCCA is executed between these attributes and the elements of correlation matrix obtained from rs-fcMRI. This procedure is the same as method B.

Sparsely selected non-diagonal elements of rs-fcMRI as the result of SCCA are used as the input of SLR, and thus the discriminator is generated (in the following, this procedure will be referred to as "method C").

Here, in SCCA, only the non-diagonal elements of correlation matrix of rs-fcMRI related to the disease/healthy label are selected. This process filters out rs-fcMRI data expressing other factors, and thus, a biomarker independent of the agency where the data is obtained can be obtained.

By performing SCCA using influences of different sites, experimental conditions and the like, it becomes possible to find which connection is related to which factor. Therefore, it becomes possible to exclude connections unrelated to the disease/healthy label, and to derive only the connections related to the disease/healthy label. This advantageously leads to "generalization" of the biomarker.

Further, by sparse method of SLR, for example, of approximately 700 connections made sparse by SCCA, twenty-one connections are used for the discriminator by the method C.

In methods B and C described above, attributes to the subject may include a label indicating whether or not a prescribed medicine has been administered, or information of dosage or duration of administration of such medicine.

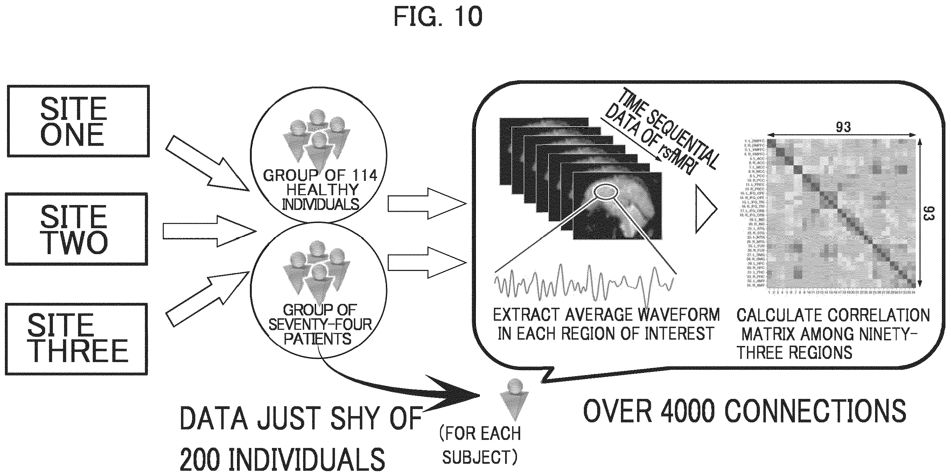

Fig. 10 shows a concept of such a procedure for generating the biomarker.

At three measurement agencies of Site one to Site three, using MRI apparatuses having different measurement performances, rs-fcMRI data of the group of 114 healthy individuals and the group of seventy-four patients were obtained, and from the resulting correlation matrix having more than 4000 connections, the discriminator is generated by the SCCA and SLR processes.

(Verification of Biomarker)

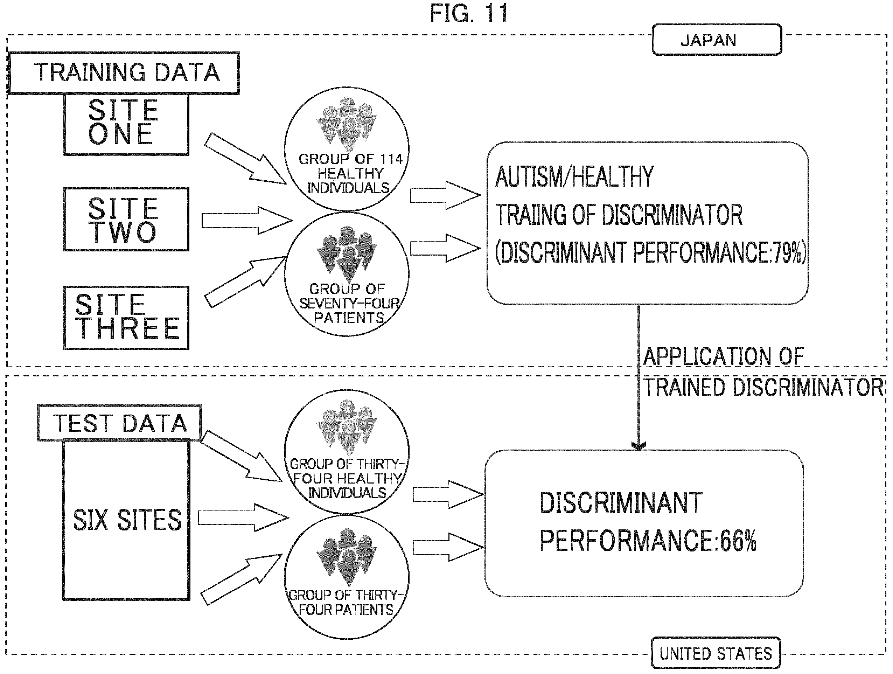

Fig. 11 shows a concept of the procedure for verifying the biomarker generated in the manner as described above.

Using the parameters for feature extraction and discriminant obtained by learning based on the data collected at Sites one to three in Japan, disease/healthy label is predicted for the data not used for the parameter learning, and consistency with the actual disease discriminant label is evaluated.

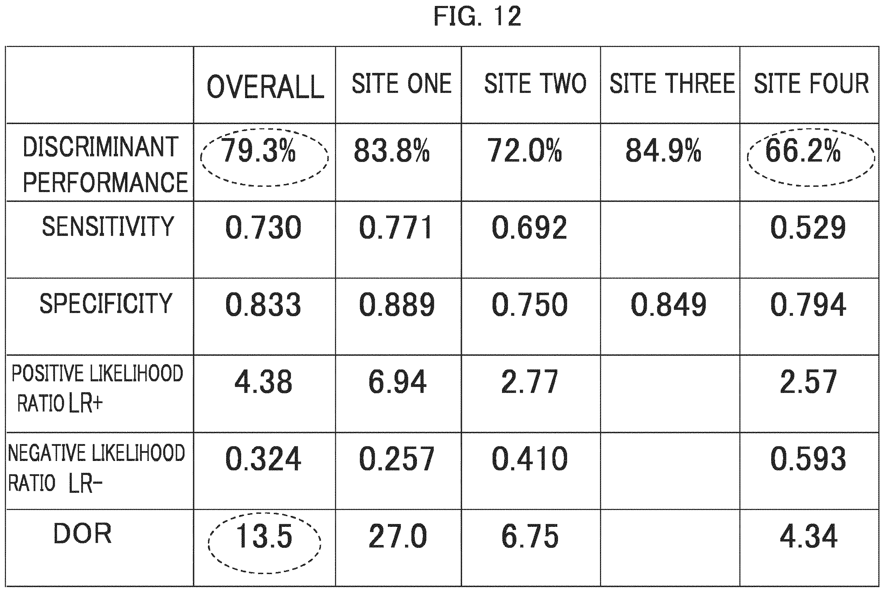

Fig. 12 shows properties of the biomarker.

As a result of consistency evaluation mentioned above, by way of example, when method C described above was used, the discriminant performance of 79% or higher at the highest was observed for the data of three different sites in Japan. When the biomarker using the same parameter was evaluated with the rs-fcMRI data measured at six sites in the United States, the discriminant performance was 66 % or higher.

In Fig. 12, Site four collectively represents the six sites in the United States.

In Fig. 12, "sensitivity" refers to the probability of correctly testing positive (having the disease) the subjects having the disease, and the "specificity" refers to the probability of correctly testing negative (not having the disease) the healthy subjects. "Positive likelihood ratio" refers to the ratio of true positive to false positive, and "negative likelihood ratio" refers to the ratio of false negative to true negative. DOR is diagnostic odds ratio, representing the ratio of sensitivity to specificity.

In Fig. 12, the generalization to the sites in the United States shows the high performance of the biomarker obtained by learning from limited data of subjects, in the sense that it goes beyond the race-by-race or country-by-country diagnostic criteria.

(Decoded Connectivity Neurofeedback)

In the foregoing, procedures have been described for obtaining data of resting state functional connectivity MRI measured for the group of healthy individuals and the group of patients, deriving correlation matrix of degree of activity of brain regions (regions of interest) for each subject, and from the non-diagonal elements of the correlation matrix, generating a biomarker for the specific disease.

In the following, a configuration of a brain activity training apparatus will be described. The apparatus uses the biomarker generated in the above-described manner to feed back brain activities to the group of patients, so as to attain the patients' correlations (connectivity) states closer to those of healthy individuals, will be described. More generally, such a brain activity training apparatus may be used not only for the training to make the state of connectivity of brain functions of patients closer to the state of connectivity of brain functions of healthy individuals considering the relation between brain functions of healthy group and patient group, but also for the training to make the state of connectivity of brain functions of a subject at present closer to the state of connectivity of brain functions of a target group.

Such a neurofeedback will be hereinafter referred to as "decoded connectivity neurofeedback."

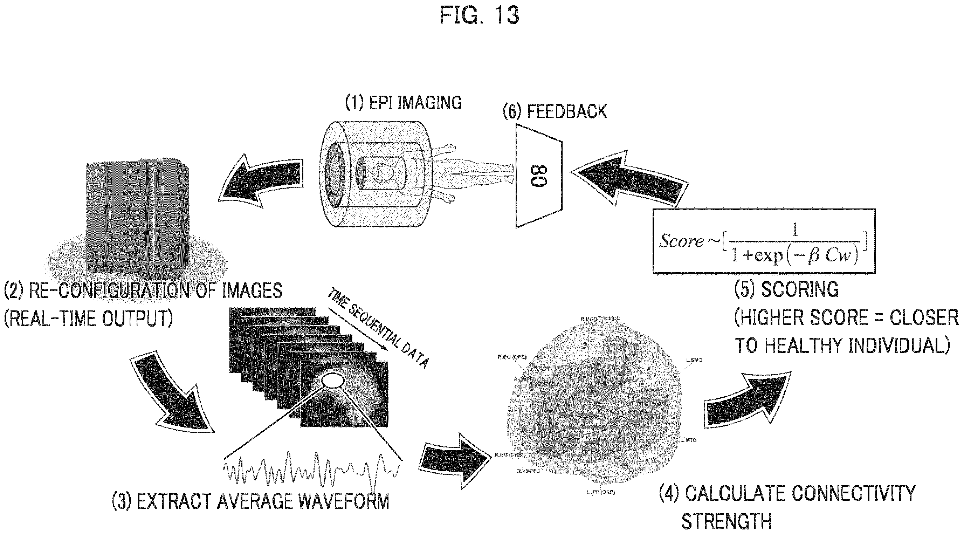

Fig. 13 shows a conceptual configuration of such a brain activity training apparatus.

The hardware configuration of the brain activity training apparatus may be the configuration of MRI apparatus 10 shown in Fig. 10 described above, for example. In the following, description will be given assuming that as the brain activity detecting apparatus for time-sequentially measuring brain activities using brain function imaging, a real-time fMRI is used.

Referring to Fig. 13, first, brain functions of a subject is measured time-sequentially for a prescribed time period, by MRI apparatus 100. For fMRI imaging, EPI (Echo-planar imaging) is executed.

Thereafter, by data processing unit 32, the taken image is re-configured on real-time basis.

Further, data processing unit 32 performs the procedure of extracting correlation matrix representing the correlation of functional connectivity, for a plurality of regions of interest. Specifically, from fMRI data of n (n: natural number) time points in the resting state measured on real-time basis, average "degree of activity" of each region of interest is calculated.

From the degree of activity obtained in this manner, data processing unit 32 calculates the correlation value (strength of connectivity) among brain regions (regions of interest).

Then, based on the calculated strength of connectivity, data processing unit 32 calculates a score, which becomes higher if it has higher degree of similarity to the strength of connectivity of a target brain function. Here, the discriminator functioning as the biomarker described above provides a value closer to one if the state is closer to that of a healthy individual (or to a subject of the target state). Therefore, by using the output of this discriminator, the score can be calculated, as will be described later. By the discriminant made by the discriminator, the result of decoding is obtained.

Data processing unit 32 displays the calculated reward value (hereinafter referred to as a score) on display 6, and thereby provides a feedback to the subject 2. The information to be fed back may be the score itself, or it may be a figure of which size changes in accordance with the magnitude of the score. Any other form may be used provided that the subject can recognize the magnitude of the score.

Thereafter, the process of score feedback from the EPI image is repeated on real-time basis for a prescribed time period.

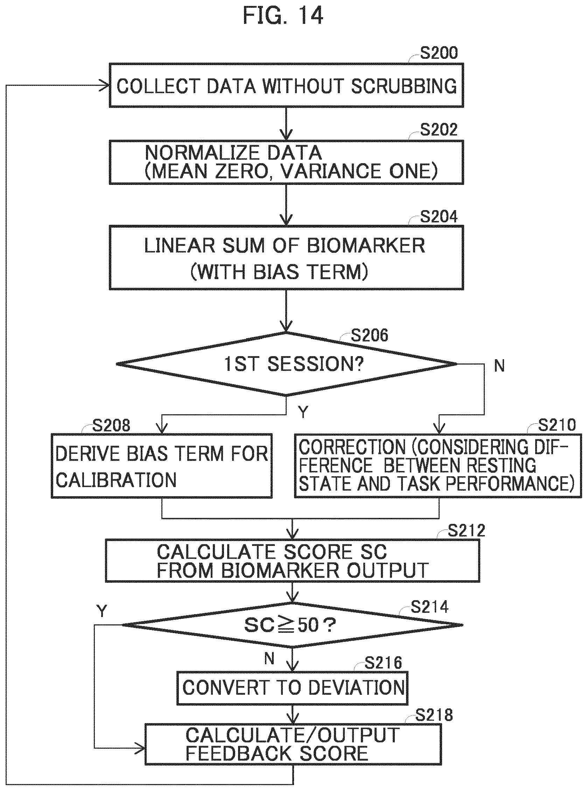

Fig. 14 is a flowchart representing a process executed by data processing unit 32 for decoded connectivity neurofeedback.

Referring to Fig. 14, first, data processing unit 32 obtains, from the data measured on real-time basis and stored in storage unit 36, "data without scrubbing" for a prescribed time period of, for example, fifteen seconds (S200).

Here, "data without scrubbing" means that pre-processing (scrubbing) for removing problematic data such as data involving movement of one's head, is not executed. Specifically, neurofeedback is an on-line process and priority is given to real-time nature and, hence, scrubbing is not performed. In contrast, collection of data for generating the biomarker described above allows off-line processing and, therefore, scrubbing is typically performed to improve data accuracy.

Thereafter, based on the collected data, data processing unit 32 calculates the correlation value of average degree of activity of brain regions (regions of interest), and normalizes the correlation value to data of mean zero, variance one (S202).

In the following, description will be given assuming that the biomarker generated in advance by the method C described above is used.

Thereafter, data processing unit 32 calculates, as the input to the biomarker (discriminator), linear weighted sum y of correlated value (S204). Here, the weight parameters used for the linear weighted sum represent the weight parameters for the discriminant for the plurality of connections (correlations) selected as a result of SLR. Specifically, they are parameters that appear as arguments of exponential function of the denominator. If there are a plurality of correlation values and a certain correlation value is selectively used for the discriminant, the value of weight parameter will be zero for the correlation value that is not selected.

Next, data processing unit 32 performs a process for calculating a bias value for calibration if the current measurement is the first session (measurement of the first one block) (S208), and, in the second and the following sessions, performs a process of correcting the measured data with the bias value in accordance with the equation below (S210).

$$\hat{y} = y + bias$$

Here, "block" refers to a minimum unit of experimental process and, as will be described later, one block includes, for example, ten trials. A "trial" includes, as will be also described later, a resting state of a prescribed time period and following task performance period and feedback period.

Specifically, in one trial, the subject undergoes:

"Resting state": the subject invokes nothing.

"Task performance period": the subject concentrates on something so as to increase the feedback score. Based on the imaging information measured in this period, the system calculates the score.

"Feedback Period": the calculated score is presented to the subject.

Specifically, in the period of fourteen seconds between the "resting state" and the "feedback period", the subject is performing the task of "concentrating" and this period is referred to as "task performance period."

Then, data processing unit 32 calculates the score SC in accordance with the equation below, using the biomarker (S212).

$$SC = 100\left(1 - \frac{1}{1 + e^{-\hat{y}}}\right)$$

Specifically, score SC corresponds to 100 times the probability that the subject is not having the disease.

If the score SC is equal to or greater than a prescribed value, for example, fifty (S214), data processing unit outputs the value as a feedback score FSC (S218).

On the other hand, if the score SC is smaller than fifty (S214), data processing unit 32 converts the value to a deviation in accordance with the equation below (S216) and outputs the result as the feedback score (S218).

$$FSC = \frac{10(SC - \mu)}{\sigma} + 50$$

Here, $\mu$ is a mean value of the first session, and $\sigma$ represents variance in the first session. Conversion to deviation is to avoid presentation of an extremely low value as the feedback value.

By the process as described above, decoded connectivity neurofeedback is executed on the subject.

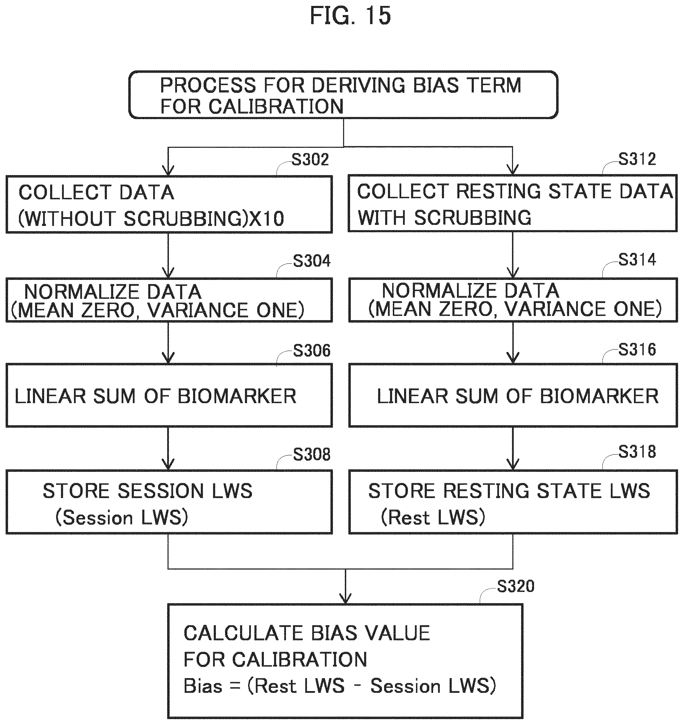

Fig. 15 is a flowchart representing a process for deriving a calibration bias term shown in Fig. 14.

Such a bias term is introduced considering the difference in linear weighted sum of correlated values input to the biomarker between the resting state and the task period for which feedback is done.

First, in the first session for the current subject, data processing unit 32 collects data of ten sets of data without scrubbing for a prescribed time period of, for example, fifteen seconds (S302).

Thereafter, data processing unit 32 calculates correlation value of average degree of activity among brain regions (regions of interest) based on the collected data, and normalizes the correlated values to data of mean zero, variance one (S304).

Further, data processing unit 32 calculates the linear weighted sum of correlated values as an input to the biomarker (discriminator) (S306).

Data processing unit 32 stores the linear weighted sum (Session LWS: Linear Weighted Sum) of correlated values for the first session in storage unit 36 (S308)

On the other hand, data processing unit 32 obtains scrubbed data of a prescribed time period, for example, of five minutes, measured in advance for the current subject (S312).

Next, based on the obtained data, data processing unit 32 calculates average correlation value of degree of activity among brain regions (regions of interest), and normalizes the correlation values to data of mean zero, variance one (S314).

Thereafter, data processing unit 32 calculates the linear weighted sum of correlation values as an input to the biomarker (discriminator) (S316).

Data processing unit 32 stores the linear weighted sum of the correlation values in the resting state (Rest LWS) in storage unit 36 (S318).

Data processing unit 32 calculates a bias value "bias" for calibration from the data stored in storage unit 36 in accordance with the equation below (S320).

$$bias = (RestLWS - SessionLWS)$$

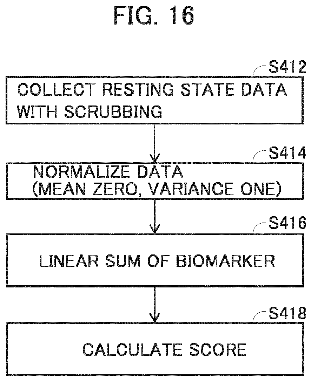

Fig. 16 is a flowchart representing a score calculating process, in accordance with the degree of connectivity among brain regions (regions of interest) in the resting state performed before and after training by decoded connectivity neurofeedback, for evaluating results of training.

Data processing unit 32 obtains scrubbed data of a prescribed time period, for example, of five minutes, measured for the subject in the resting state and stored in storage unit 36 (S412).

Thereafter, based on the obtained data, data processing unit 32 calculates average correlation value of degree of activity among brain regions (regions of interest), and normalizes the correlation values to data of mean zero, variance one (S414).

Further, data processing unit 32 calculates the linear weighted sum of correlation values as input to the biomarker (discriminator) (S416).

Based on the output of biomarker, data processing unit 32 calculates a score SC in the similar manner as shown in Fig. 14 (S418).

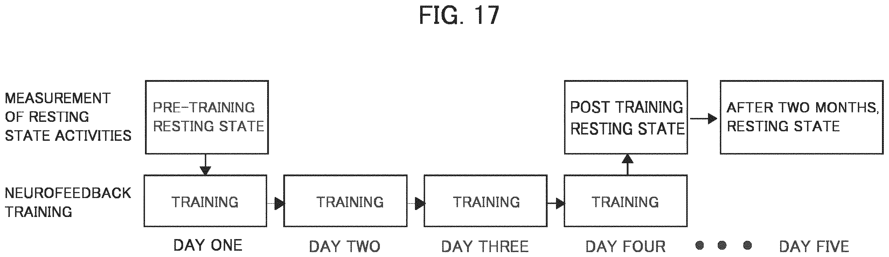

Fig. 17 shows an example of a training sequence in the decoded connectivity neurofeedback.

Referring to Fig. 17, first, on Day one, the subject has his/her pre-training brain activities in the resting state measured in accordance with the flowchart of Fig. 16.

On Day one, thereafter, the subject undergoes the training by the decoded connectivity neurofeedback, in accordance with the flow shown in Fig. 14.

Thereafter, on Day two and Day three, the subject undergoes the training by the decoded connectivity neurofeedback, in accordance with the flow shown in Fig. 14.

On Day four (last day), the subject undergoes the training by the decoded connectivity neurofeedback, in accordance with the flow shown in Fig. 14, and thereafter, post-training brain activities in the resting state are measured in accordance with the flowchart of Fig. 16. The number of days for the training may be smaller than or larger than the example here.

Further, after a prescribed period of time, for example, after two months, brain activities in the resting state are measured in accordance with the flowchart of Fig. 16.

In the following, an example in which correlation between left lateral parietal area ILP and left primary motor cortex IM1 of the default mode network was changed by training will be described, to verify the effectiveness of decoded connectivity neurofeedback.

By way of example, it is expected that by the correlation between lateral parietal area LP and primary motor cortex M1, reaction time to word-stroop problem between the color of a word expressed as letters and the meaning of the word would change. Specifically, the time required for naming the color, when the name of a color is in a color (blue) not denoted by the name (red), as in the case when a word "red" is written in blue (referred to as Stroop stimulus), is expected to change.

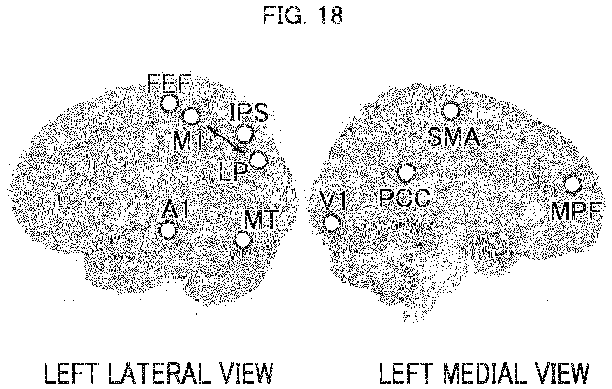

Fig. 18 shows in-brain positions of left lateral parietal area ILP and left primary motor cortex IM1.

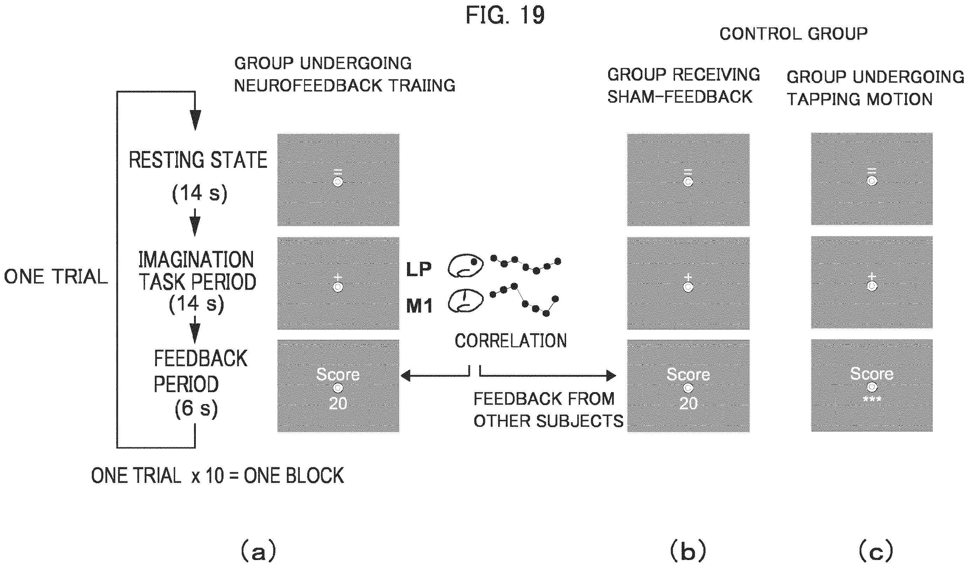

Fig. 19 shows a training sequence of the decoded connectivity neurofeedback.

As shown in Fig. 19, a group of subjects (1st group) undergoes fourteen seconds of resting state, views a prescribed presented image (an image designating an imagination task) for fourteen seconds, and thereafter, receives feedback of their scores, in one trial. This trial repeated, for example, ten times, is referred to as one block. A number of blocks of such feedback is repeated in each day.

On the other hand, for verifying the decoded connectivity neurofeedback, another group of subjects (2nd group) receives presentation of feedback information generated by the brain activities measured for other subjects.

A still another group of subjects (3rd group) receives presentation of an image (image designating imagination of tapping motion) similar to the first group, except that the scores are not presented.

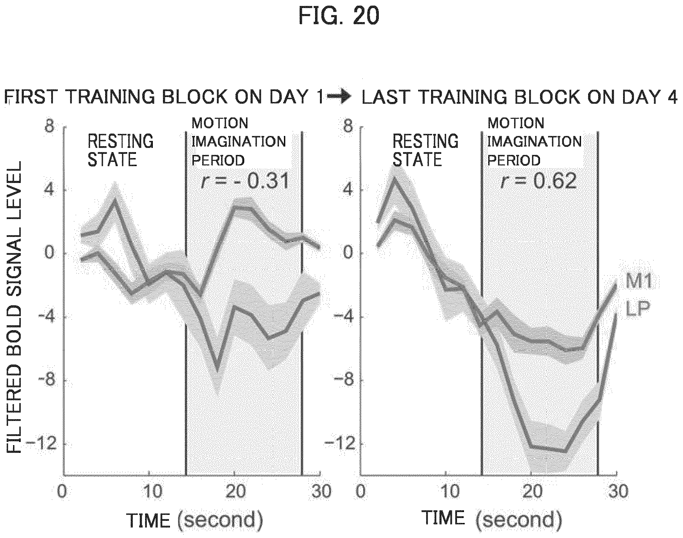

Fig. 20 shows changes between initial stage and terminal stage of brain function correlation (connectivity) training of a subject of the first group.

As shown in Fig. 20, in the first training block of Day one, the correlation between left lateral parietal area ILP and left primary motor cortex IM1 of this subject was negative, as indicated by a correlation coefficient r = -0.31. In contrast, in the last training block of Day four, the correlation between lateral parietal area LP and primary motor cortex M1 was changed to positive, as indicated by the correlation coefficient r = 0.62.

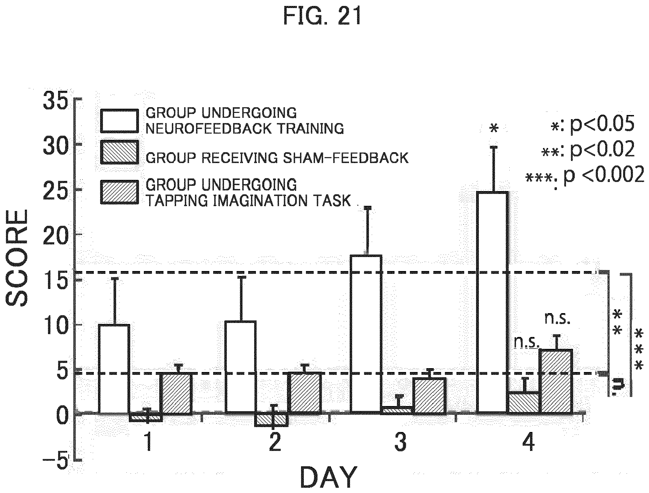

Fig. 21 shows results of measurement of brain activity in the resting state after the last training of each of the first to third groups.

As can be seen from Fig. 21, the score of the first group that went through the decoded connectivity neurofeedback significantly improved.

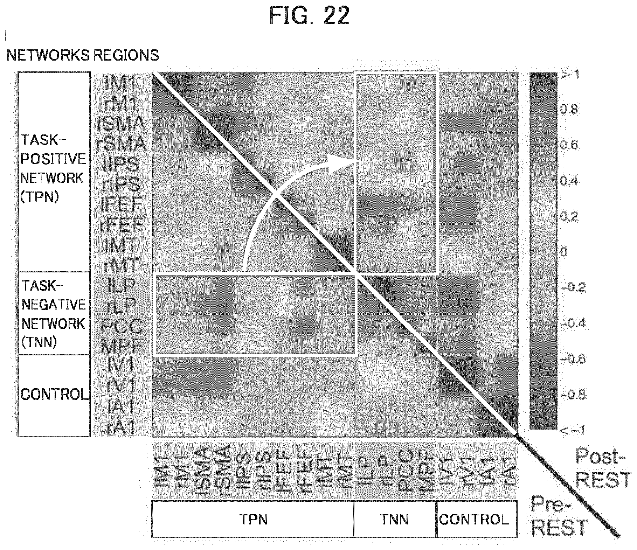

Fig. 22 shows brain activity correlation by the measurement of brain activity in the resting state before training and brain activity correlation by the measurement of brain activity in the resting state after training shown in Fig. 17.

It can be seen that the correlation changed such that regions that had a negative correlation before training came to have a positive correlation.

It was experimentally confirmed that such a change in correlation was maintained after two months.

(Application Example to a Subject Diagnosed as Having Autism)

In the following, an example of decoded connectivity neurofeedback actually applied to a subject who is diagnosed as having autism, under the direction and supervision of a doctor, will be described.

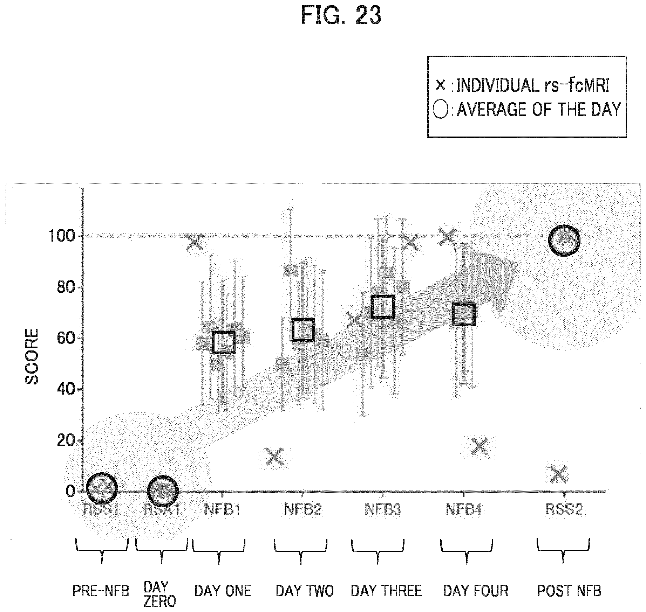

Fig. 23 shows changes in scores when decoded connectivity neurofeedback is performed on a subject diagnosed as having autism.

Referring to Fig. 23, for the neurofeedback, first, resting-state functional connectivity MRI (rs-fcMRI) of the subject diagnosed as having autism was measured and scores related to autism are calculated using the biomarker described above, before conducting neurofeedback (hereinafter simply referred to as NFB). In Fig. 23, circles each represent an average score of that day, and squares each represent a score during the decoded connectivity neurofeedback.

Then, on Day zero, or immediately before starting the decoded connectivity neurofeedback, the score is confirmed again by rs-fcMRI.

On Day zero, the score SC of the subject is zero, that is, the output of biomarker represents determination as having autism.

Then, in accordance with the procedure described above, decoded connectivity neurofeedback is performed on Day one to Day four.

A prescribed time period after the decoded connectivity neurofeedback, the score was calculated by measuring again with rs-fcMRI. It was found that the score reached almost 100, which corresponds to the determination by the biomarker output of "not having a disease," after the prescribed time period.

Though further study is indispensable for clinical application, this result suggests a possibility that the decoded connectivity neurofeedback is applicable to a therapy of autism.

As described above, by using the method of decoded connectivity neurofeedback in accordance with the present embodiment, it is possible to change through training the correlation of connections among brain regions by utilizing the correlation of connections among brain regions measured by real-time fMRI.

In the foregoing description, it is assumed that real-time fMRI is used as the brain activity detecting apparatus for time-sequentially measuring brain activities by functional brain imaging. It is noted, however, that any of the fMRI described above, a magnetoencephalography, a near-infrared spectroscopy (NIRS), an electroencephalography or a combination of any of these may be used as the brain activity detecting apparatus. Regarding such a combination, it is noted that fMRI and NIRS detect signals related to change in blood flow in the brain, and have high spatial resolution. On the other hand, magnetoencephalography and electroencephalography are characterized in that they have high temporal resolution, for detecting change in electromagnetic field associated with the brain activities. Therefore, if fMRI and the magnetoencephalography are combined, brain activities can be measured with both spatially and temporally high resolutions. Alternatively, by combining NIRS and the electroencephalography, a system for measuring brain activities with both spatially and temporally high resolutions can also be implemented in a small, portable size.

By the configuration as described above, it becomes possible to realize a brain activity training apparatus that changes correlation of connections among brain regions utilizing correlation among brain regions measured by functional brain imaging as feedback information.

In the foregoing, an example has been described in which a "disease discriminant label" is included as an attribute of a subject, and by generating a discriminator through machine learning, the discriminator is caused to function as a biomarker. The present invention, however, is not necessarily limited to such an example. Provided that a group of subjects whose results of measurements are to be obtained as the object of machine learning is classified into a plurality of classes in advance by an objective method, the correlation of degree of activity (connectivity) among brain regions (regions of interest) of the subjects is measured and a discriminator can be generated for classification by machine learning using the measured results, the present invention may be used for other discrimination.

Therefore, by evaluating beforehand whether a specific "training pattern" by the brain activity training apparatus is useful for improving health of a subject and utilizing the result, a brain activity training apparatus for improving health can be implemented. Further, by objectively evaluating beforehand whether a specific "training pattern" by the brain activity training apparatus is useful for a subject to attain healthier state, a brain activity training apparatus to attain better condition before onset of a disease can be realized.

The embodiments as have been described here are mere examples and should not be interpreted as restrictive. The scope of the present invention is determined by each of the claims

REFERENCE SIGNS LIST

2 subject, 6 display, 10 MRI apparatus, 11 magnetic field applying mechanism, 12 static magnetic field generating coil, 14 magnetic field gradient generating coil, 16 RF irradiating unit, 18 bed, 20 receiving coil, 21 driving unit, 22 static magnetic field power source, 24 magnetic field gradient power source, 26 signal transmitting unit, 28 signal receiving unit, 30 bed driving unit, 32 data processing unit, 36 storage unit, 38 display unit, 40 input unit, 42 control unit, 44 interface unit, 46 data collecting unit, 48 image processing unit, 50 network interface.

**Claims**

1.  A brain activity training apparatus (10), comprising:

    a brain activity detecting device (20) for time-sequentially detecting signals indicative of brain activities at a plurality of prescribed regions in a brain of a first subject (2); and
    a storage device (36) for storing information that specifies a discriminator generated from signals measured in advance by time-sequentially measuring signals indicative of the brain activities at said plurality of prescribed regions in a brain of each of a plurality of second subjects (2) different from said first subject, said discriminator executing a discrimination of a target attribute among attributes of said second subjects (2) by contraction expression extracted, from correlations of brain activities in said plurality of prescribed regions, each correlation relating to a different pair of prescribed regions, commonly with respect to at least attributes of said plurality of second subjects;
    said brain activity training apparatus (10) further comprising:

    a presenting device (6); and
    a processing device (32); wherein
    said processing device (32) is configured to

    i) calculate a correlation of brain activities for each pair of prescribed regions, based on signals detected by said brain activity detecting device,
    ii) based on said calculated correlations, by said discriminator specified by the information stored in said storage device, calculate a reward value in accordance with degree of similarity of said calculated correlations to target correlations corresponding to said target attribute, and
    iii) present information indicative of magnitude of said reward value to said subject by said presenting device (6).

2.  The brain activity training apparatus (10) according to claim 1, wherein
    said signals measured in advance from said plurality of second subjects (2) are measured by a plurality of brain activity measuring devices (10); and
    said contraction expression used by said discriminator is a contraction expression extracted, by variable selection from correlations of brain activities among said plurality of prescribed regions, commonly with respect to measuring conditions of said plurality of brain activity measuring devices (10), and attributes of said plurality of subjects.

3. The brain activity training apparatus (10) according to claim 1 or 2, wherein
said discriminator is generated by regression of performing further variable selection on said extracted contraction expression.

4. The brain activity training apparatus (10) according to claim 1, wherein
said discriminator is generated by sparse logistic regression on sparse non-diagonal elements and a target attribute of said second subjects, said sparse non-diagonal elements being made sparse based on a result obtained by regularized canonical correlation analysis with respect to non-diagonal elements of correlation matrix of brain activities at said plurality of prescribed regions of said second subjects (2) and said attributes of said second subjects; and
an input to said discriminator is a linear weighted sum of said non-diagonal elements of said first subject (2) corresponding to the result of said regularized canonical correlation analysis.

5. The brain activity training apparatus (10) according to claim 2, wherein
said discriminator is generated by sparse logistic regression on sparse non-diagonal elements and a target attribute of said second subjects, said sparse non-diagonal elements being made sparse based on a result obtained by regularized canonical correlation analysis with respect to non-diagonal elements of correlation matrix of brain activities at said plurality of prescribed regions of said second subjects (2) and said attributes of said second subjects; and
an input to said discriminator is a non-diagonal element selected as related to said target attribute, from said non-diagonal elements based on a result of said regularized canonical correlation analysis.

6. The brain activity training apparatus (10) according to claim 1, wherein
said brain activity detecting device (20) includes a brain activity detecting device (20) for picking-up a resting-state functional connectivity magnetic resonance image.

7. The brain activity training apparatus (10) according to claim 4 or 5, wherein
said regularized canonical correlation analysis is canonical correlation analysis with L1 regularization.

8. A brain activity training system (10), comprising:

a brain activity detecting device (20) for time-sequentially detecting signals indicative of brain activities at a plurality of prescribed regions in a brain of a first subject; and
discriminator generating means (32) for generating a discriminator from signals measured in advance by said brain activity detecting device (20) by time-sequentially measuring signals indicative of the brain activities at said plurality of prescribed regions in a brain of each of a plurality of second subjects (2) different from said first subject, said discriminator generating means (32) extracting contraction expression common to at least attributes of said plurality of second subjects (2) from correlations of brain activities in said plurality of prescribed regions, each correlation relating to a different pair of prescribed regions, and generates a discriminator for the extracted contraction expression with respect to a target attribute among said attributes of said second subjects;
said brain activity training system (10) further comprising:

a storage device (36) for storing information that specifies said discriminator;
a presenting device (6); and
a processing device (32); wherein
said processing device (32) is configured to

i) calculate a correlation of brain activities for each pair of prescribed regions, based on signals detected by said brain activity detecting device,
ii) based on said calculated correlations, by a discriminant process by said discriminator specified by the information stored in said storage device, calculate a reward value in accordance with degree of similarity of said calculated correlations to target correlations corresponding to said target attribute, and
iii) present information indicative of magnitude of said reward value to said subject by said presenting device (6).

9. The brain activity training system (10) according to claim 8, wherein

said brain activity detecting device (20) includes a plurality of brain activity measuring devices (10); and
said discriminator generating means (32) includes extracting means for extracting, by variable selection from correlations of brain activities among said plurality of prescribed regions, a contraction expression common to measuring conditions of said plurality of brain activity measuring devices (10), and attributes of said plurality of subjects.

10. The brain activity training system (10) according to claim 8 or 9, wherein
said discriminator generating means (32) includes regression means for generating said discriminator by regression of performing further variable selection on said extracted contraction expression.

11. The brain activity training system (10) according to claim 9, wherein
said extracting means includes correlation analyzing means for calculating a correlation matrix of activities at said plurality of prescribed regions from the signals detected by said brain activity detecting device, executing regularized canonical correlation analysis between attributes of said subjects and non-diagonal elements of said correlation matrix and thereby for extracting said contracted expression.

12. The brain activity training system (10) according to claim 10, wherein
said regression means includes regression analysis means for generating a discriminator by sparse logistic regression on a result of said regularized canonical correlation analysis and the attributes of said subjects.

13. The brain activity training system (10) according to claim 9, wherein
said plurality of brain activity measuring devices (10) are devices for time-sequentially measuring brain activities by functional brain imaging installed at a plurality of different locations, respectively.

14. The brain activity training system (10) according to claim 8, wherein
said discriminant process is discrimination of a disease label indicating whether said first subject (2) is healthy or a patient of a neurological/mental disorder.

15. The brain activity training system (10) according to claim 9, wherein
the attributes of said subjects include a disease label indicating whether said subject is healthy or a patient of a neurological/mental disorder, a label indicating individual nature of said subject, and information characterizing measurement by said brain activity detecting device; and
said discriminant process is discrimination of a disease label indicating whether said first subject (2) is healthy or a patient of a neurological/mental disorder.

16. The brain activity training system (10) according to claim 11, wherein
said regularized canonical correlation analysis is canonical correlation analysis with L1 regularization.

17. The brain activity training system (10) according to claim 9, wherein
said brain activity measuring device picks up a resting-state functional connectivity magnetic resonance image.

**Patentansprüche**

1. Apparat (10) zum Trainieren der Hirnaktivität, aufweisend:

eine eine Hirnaktivität detektierende Vorrichtung (20), um Signale zeitlich nacheinander zu detektieren, die Hirnaktivitäten an einer Vielzahl vorgeschriebener Bereiche in einem Gehirn einer ersten Testperson (2) anzeigen; und
eine Speichervorrichtung (36), um eine Information zu speichern, die einen Diskriminator spezifiziert, der aus Signalen erzeugt wird, die vorher gemessen werden, indem Signale zeitlich nacheinander gemessen werden, die die Hirnaktivitäten an der Vielzahl vorgeschriebener Bereiche in einem Gehirn von jedem einer Vielzahl zweiter Testpersonen (2), die von der ersten Testperson verschieden sind, anzeigen, wobei der Diskriminator eine Unterscheidung eines Zielattributs unter Attributen der zweiten Testpersonen (2) mittels eines Kontraktionsausdrucks ausführt, der aus Korrelationen von Hirnaktivitäten in der Vielzahl vorgeschriebener Bereiche, wobei sich jede Korrelation auf ein unterschiedliches Paar vorgeschriebener Bereiche bezieht, gemeinsam in Bezug auf zumindest Attribute der Vielzahl zweiter Testpersonen extrahiert wird;
wobei der Apparat (10) zum Trainieren der Hirnaktivität ferner aufweist:

eine Präsentationsvorrichtung (6); und
eine Verarbeitungsvorrichtung (32); wobei
die Verarbeitungsvorrichtung (32) dafür konfiguriert ist,

i) eine Korrelation von Hirnaktivitäten für jedes Paar vorgeschriebener Bereiche basierend auf Signalen zu berechnen, die durch die eine Hirnaktivität detektierende Vorrichtung detektiert werden,

ii) basierend auf den berechneten Korrelationen durch den Diskriminator, der durch die in der Speichervorrichtung gespeicherte Information spezifiziert ist, gemäß einem Grad einer Ähnlichkeit der berechneten Korrelationen zu Zielkorrelationen entsprechend dem Zielattribut einen Reward-Wert zu berechnen und

iii) eine Information, die eine Größe des Reward-Werts anzeigt, der Testperson mittels der Präsentationsvorrichtung (6) zu präsentieren.

2. Apparat (10) zum Trainieren der Hirnaktivität nach Anspruch 1, wobei
die vorher von der Vielzahl zweiter Testpersonen (2) gemessenen Signale mittels einer Vielzahl von eine Hirnaktivität messenden Vorrichtungen (10) gemessen werden; und
der vom Diskriminator genutzte Kontraktionsausdruck ein Kontraktionsausdruck ist, der durch eine Variablenauswahl aus Korrelationen von Hirnaktivitäten unter der Vielzahl vorgeschriebener Bereiche gemeinsam in Bezug auf Messbedingungen der Vielzahl von eine Hirnaktivität messenden Vorrichtungen (10) und Attribute der Vielzahl von Testpersonen extrahiert wird.

3. Apparat (10) zum Trainieren der Hirnaktivität nach Anspruch 1 oder 2, wobei
der Diskriminator durch Regression einer Durchführung einer weiteren Variablenauswahl an dem extrahierten Kontraktionsausdruck erzeugt wird.

4. Apparat (10) zum Trainieren der Hirnaktivität nach Anspruch 1, wobei
der Diskriminator durch eine logistische Sparse-Regression an nicht-diagonalen Sparse-Elementen und ein Zielattribut der zweiten Testpersonen erzeugt wird, wobei die nicht-diagonalen Sparse-Elemente basierend auf einem Ergebnis ausgedünnt werden, das durch eine regularisierte kanonische Korrelationsanalyse in Bezug auf nicht-diagonale Elemente einer Korrelationsmatrix von Hirnaktivitäten an der Vielzahl vorgeschriebener Bereiche der zweiten Testpersonen (2) und die Attribute der zweiten Testpersonen erhalten wird; und
eine Eingabe in den Diskriminator eine lineare gewichtete Summe der nicht-diagonalen Elemente der ersten Testperson (2) entsprechend dem Ergebnis der regularisierten kanonischen Korrelationsanalyse ist.

5. Apparat (10) zum Trainieren der Hirnaktivität nach Anspruch 2, wobei
der Diskriminator durch eine logistische Sparse-Regression an nicht-diagonalen Sparse-Elementen und ein Zielattribut der zweiten Testpersonen erzeugt wird, wobei die nicht-diagonalen Sparse-Elemente basierend auf einem Ergebnis ausgedünnt werden, das durch eine regularisierte kanonische Korrelationsanalyse in Bezug auf nicht-diagonale Elemente einer Korrelationsmatrix von Hirnaktivitäten an der Vielzahl vorgeschriebener Bereiche der zweiten Testpersonen (2) und die Attribute der zweiten Testpersonen erhalten wird; und
eine Eingabe in den Diskriminator ein nicht-diagonales Element ist, das als mit dem Zielattribut zusammenhängend unter den nicht-diagonalen Elementen basierend auf einem Ergebnis der regularisierten kanonischen Korrelationsanalyse ausgewählt wird.

6. Apparat (10) zum Trainieren der Hirnaktivität nach Anspruch 1, wobei
die eine Hirnaktivität detektierende Vorrichtung (20) eine eine Hirnaktivität detektierende Vorrichtung (20) zum Aufnehmen eines Magnetresonanzbildes einer funktionalen Konnektivität im Ruhezustand enthält.

7. Apparat (10) zum Trainieren der Hirnaktivität nach Anspruch 4 oder 5, wobei
die regularisierte kanonische Korrelationsanalyse eine kanonische Korrelationsanalyse mit einer L1-Regularisierung ist.

8. System (10) zum Trainieren der Hirnaktivität, aufweisend:

eine eine Hirnaktivität detektierende Vorrichtung (20), um Signale zeitlich nacheinander zu detektieren, die Hirnaktivitäten an einer Vielzahl vorgeschriebener Bereiche in einem Gehirn einer ersten Testperson anzeigen; und
ein einen Diskriminator erzeugendes Mittel (32), um einen Diskriminator aus Signalen zu erzeugen, die durch die eine Hirnaktivität detektierende Vorrichtung (20) vorher gemessen werden, indem Signale zeitlich nacheinander gemessen werden, die die Hirnaktivitäten an der Vielzahl vorgeschriebener Bereiche in einem Gehirn von jedem einer Vielzahl zweiter Testpersonen (2), die von der ersten Testperson verschieden sind, anzeigen, wobei das einen Diskriminator erzeugende Mittel (32) einen Kontraktionsausdruck gemeinsam mit zumindest Attributen der Vielzahl zweiter Testpersonen (2) aus Korrelationen von Hirnaktivitäten in der Vielzahl vorgeschriebener Bereiche, wobei sich jede Korrelation auf ein unterschiedliches Paar vorgeschriebener Bereiche bezieht, extrahiert und einen Diskriminator für den extrahierten Kontraktionsausdruck in Bezug auf ein Zielattribut unter den Attributen der zweiten Testpersonen erzeugt;

wobei das System (10) zum Trainieren der Hirnaktivität ferner aufweist:

eine Speichervorrichtung (36), um eine Information, die den Diskriminator spezifiziert, zu speichern;
eine Präsentationsvorrichtung (6); und
eine Verarbeitungsvorrichtung (32); wobei
die Verarbeitungsvorrichtung (32) dafür konfiguriert ist,

i) eine Korrelation von Hirnaktivitäten für jedes Paar vorgeschriebener Bereiche basierend auf Signalen zu berechnen, die durch die eine Hirnaktivität detektierende Vorrichtung detektiert werden,
ii) basierend auf den berechneten Korrelationen mittels eines Unterscheidungsprozesses durch den Diskriminator, der durch die in der Speichervorrichtung gespeicherte Information spezifiziert ist, gemäß einem Grad einer Ähnlichkeit der berechneten Korrelationen zu Zielkorrelationen entsprechend dem Zielattribut einen Reward-Wert zu berechnen und
iii) eine Information, die eine Größe des Reward-Werts anzeigt, der Testperson mittels der Präsentationsvorrichtung (6) zu präsentieren.

9. System (10) zum Trainieren der Hirnaktivität nach Anspruch 8, wobei
die eine Hirnaktivität detektierende Vorrichtung (20) eine Vielzahl von eine Hirnaktivität messenden Vorrichtungen (10) enthält; und
das einen Diskriminator erzeugende Mittel (32) ein Extrahiermittel enthält, um durch eine Variablenauswahl aus Korrelationen von Hirnaktivitäten unter der Vielzahl vorgeschriebener Bereiche einen Kontraktionsausdruck gemeinsam mit Messbedingungen der Vielzahl von eine Hirnaktivität messenden Vorrichtungen (10) und Attributen der Vielzahl von Testpersonen zu extrahieren.

10. System (10) zum Trainieren der Hirnaktivität nach Anspruch 8 oder 9, wobei
das einen Diskriminator erzeugende Mittel (32) ein Regressionsmittel enthält, um den Diskriminator mittels Regression einer Durchführung einer weiteren Variablenauswahl an dem extrahierten Kontraktionsausdruck zu erzeugen.

11. System (10) zum Trainieren der Hirnaktivität nach Anspruch 9, wobei
das Extrahiermittel ein eine Korrelation analysierendes Mittel enthält, um eine Korrelationsmatrix von Aktivitäten an der Vielzahl vorgeschriebener Bereiche aus den durch die eine Hirnaktivität detektierende Vorrichtung detektierten Signalen zu berechnen, eine regularisierte kanonische Korrelationsanalyse zwischen Attributen der Testpersonen und nicht-diagonalen Elementen der Korrelationsmatrix auszuführen und dadurch den kontrahierten Ausdruck zu extrahieren.

12. System (10) zum Trainieren der Hirnaktivität nach Anspruch 10, wobei
das Regressionsmittel ein Regressionsanalysemittel enthält, um einen Diskriminator durch eine logistische Sparse-Regression an einem Ergebnis der regularisierten kanonischen Korrelationsanalyse und die Attribute der Testpersonen zu erzeugen.

13. System (10) zum Trainieren der Hirnaktivität nach Anspruch 9, wobei
die Vielzahl von eine Hirnaktivität messenden Vorrichtungen (10) Vorrichtungen sind, um Hirnaktivitäten durch eine funktionale Hirnabbildung zeitlich nacheinander zu messen, die jeweils an einer Vielzahl verschiedener Stellen installiert sind.

14. System (10) zum Trainieren der Hirnaktivität nach Anspruch 8, wobei
der Unterscheidungsprozess eine Unterscheidung eines Krankheitszeichens ist, das anzeigt, ob die erste Testperson (2) gesund oder ein Patient mit einer neurologischen/mentalen Erkrankung ist.

15. System (10) zum Trainieren der Hirnaktivität nach Anspruch 9, wobei

die Attribute der Testpersonen ein Krankheitszeichen, das anzeigt, ob die Testperson gesund oder ein Patient mit einer neurologischen/mentalen Erkrankung ist, ein Zeichen, das die individuelle Natur der Testperson anzeigt, und eine Information einschließen, die eine Messung durch die eine Hirnaktivität detektierende Vorrichtung kennzeichnet; und

der Unterscheidungsprozess eine Unterscheidung eines Krankheitszeichens ist, das anzeigt, ob die erste Testperson (2) gesund oder ein Patient mit einer neurologischen/mentalen Erkrankung ist.

**16.** System (10) zum Trainieren der Hirnaktivität nach Anspruch 11, wobei
die regularisierte kanonische Korrelationsanalyse eine kanonische Korrelationsanalyse mit einer L1-Regularisierung ist.

**17.** System (10) zum Trainieren der Hirnaktivität nach Anspruch 9, wobei
die eine Hirnaktivität detektierende Vorrichtung ein Magnetresonanzbild einer funktionalen Konnektivität im Ruhezustand aufnimmt.


**Revendications**

**1.** Appareil d'entraînement à l'activité cérébrale (10), comprenant :

un dispositif de détection d'activité cérébrale (20) pour détecter séquentiellement dans le temps des signaux indicatifs d'activités cérébrales au niveau d'une pluralité de régions prescrites dans un cerveau d'un premier sujet (2) ; et
un dispositif de stockage (36) pour stocker des informations qui spécifient un discriminateur généré à partir de signaux mesurés à l'avance en mesurant séquentiellement dans le temps des signaux indicatifs des activités cérébrales au niveau de ladite pluralité de régions prescrites dans un cerveau de chacun d'une pluralité de deuxièmes sujets (2) différents dudit premier sujet, ledit discriminateur exécutant une discrimination d'un attribut cible parmi des attributs desdits deuxièmes sujets (2) par une expression de contraction extraite, à partir de corrélations d'activités cérébrales dans ladite pluralité de régions prescrites, chaque corrélation se rapportant à une paire différente de régions prescrites, communément par rapport à au moins des attributs de ladite pluralité de deuxièmes sujets ;
ledit appareil d'entraînement à l'activité cérébrale (10) comprenant en outre :

un dispositif de présentation (6) ; et
un dispositif de traitement (32) ; dans lequel
ledit dispositif de traitement (32) est configuré pour

i) calculer une corrélation d'activités cérébrales pour chaque paire de régions prescrites, sur la base des signaux détectés par ledit dispositif de détection d'activité cérébrale,
ii) sur la base desdites corrélations calculées, par ledit discriminateur spécifié par les informations stockées dans ledit dispositif de stockage, calculer une valeur de récompense en fonction du degré de similarité desdites corrélations calculées avec des corrélations cibles correspondant audit attribut cible, et
iii) présenter des informations indicatives d'une ampleur de ladite valeur de récompense audit sujet par ledit dispositif de présentation (6).

**2.** Appareil d'entraînement à l'activité cérébrale (10) selon la revendication 1, dans lequel
lesdits signaux mesurés à l'avance à partir de ladite pluralité de deuxièmes sujets (2) sont mesurés par une pluralité de dispositifs de mesure d'activité cérébrale (10) ; et
ladite expression de contraction utilisée par ledit discriminateur est une expression de contraction extraite, par sélection variable à partir de corrélations d'activités cérébrales parmi ladite pluralité de régions prescrites, communément par rapport à des conditions de mesure de ladite pluralité de dispositifs de mesure d'activité cérébrale (10), et des attributs de ladite pluralité de sujets.

**3.** Appareil d'entraînement à l'activité cérébrale (10) selon la revendication 1 ou 2, dans lequel
ledit discriminateur est généré par une régression de réalisation d'une sélection variable supplémentaire sur ladite expression de contraction extraite.

**4.** Appareil d'entraînement à l'activité cérébrale (10) selon la revendication 1, dans lequel
ledit discriminateur est généré par régression logistique clairsemée sur des éléments non diagonaux clairsemés et un attribut cible desdits deuxièmes sujets, lesdits éléments non diagonaux clairsemés étant rendus clairsemés sur la base d'un résultat obtenu par analyse de corrélation canonique régularisée par rapport à des éléments non diagonaux d'une matrice de corrélation d'activités cérébrales au niveau de ladite pluralité de régions prescrites desdits deuxièmes sujets (2) et desdits attributs desdits deuxièmes sujets ; et
une entrée audit discriminateur est une somme pondérée linéaire desdits éléments non diagonaux dudit premier sujet (2) correspondant au résultat de ladite analyse de corrélation canonique régularisée.

**5.** Appareil d'entraînement à l'activité cérébrale (10) selon la revendication 2, dans lequel
ledit discriminateur est généré par régression logistique clairsemée sur des éléments non diagonaux clairsemés et un attribut cible desdits deuxièmes sujets, lesdits éléments non diagonaux clairsemés étant rendus clairsemés sur la base d'un résultat obtenu par analyse de corrélation canonique régularisée par rapport à des éléments non diagonaux d'une matrice de corrélation d'activités cérébrales au niveau de ladite pluralité de régions prescrites desdits deuxièmes sujets (2) et desdits attributs desdits deuxièmes sujets ; et
une entrée audit discriminateur est un élément non diagonal sélectionné comme étant lié audit attribut cible, à partir desdits éléments non diagonaux sur la base d'un résultat de ladite analyse de corrélation canonique régularisée.

**6.** Appareil d'entraînement à l'activité cérébrale (10) selon la revendication 1, dans lequel
ledit dispositif de détection d'activité cérébrale (20) comporte un dispositif de détection d'activité cérébrale (20) pour prendre une image de résonance magnétique de connectivité fonctionnelle d'état de repos.

**7.** Appareil d'entraînement à l'activité cérébrale (10) selon la revendication 4 ou 5, dans lequel
ladite analyse de corrélation canonique régularisée est une analyse de corrélation canonique avec une régularisation L1.

**8.** Système d'entraînement à l'activité cérébrale (10), comprenant :

un dispositif de détection d'activité cérébrale (20) pour détecter séquentiellement dans le temps des signaux indicatifs d'activités cérébrales au niveau d'une pluralité de régions prescrites dans un cerveau d'un premier sujet ; et
un moyen de génération de discriminateur (32) pour générer un discriminateur à partir de signaux mesurés à l'avance par ledit dispositif de détection d'activité cérébrale (20) en mesurant séquentiellement dans le temps des signaux indicatifs des activités cérébrales au niveau de ladite pluralité de régions prescrites dans un cerveau de chacun d'une pluralité de deuxièmes sujets (2) différents dudit premier sujet, ledit moyen de génération de discriminateur (32) extrayant une expression de contraction commune à au moins des attributs de ladite pluralité de deuxièmes sujets (2) à partir de corrélations d'activités cérébrales dans ladite pluralité de régions prescrites, chaque corrélation se rapportant à une paire différente de régions prescrites, et génère un discriminateur pour l'expression de contraction extraite par rapport à un attribut cible parmi lesdits attributs desdits deuxièmes sujets ;
ledit système d'entraînement à l'activité cérébrale (10) comprenant en outre :

un dispositif de stockage (36) pour stocker des informations qui spécifient ledit discriminateur ;
un dispositif de présentation (6) ; et
un dispositif de traitement (32) ; dans lequel
ledit dispositif de traitement (32) est configuré pour

i) calculer une corrélation d'activités cérébrales pour chaque paire de régions prescrites, sur la base des signaux détectés par ledit dispositif de détection d'activité cérébrale,
ii) sur la base desdites corrélations calculées, par un processus discriminant par ledit discriminateur spécifié par les informations stockées dans ledit dispositif de stockage, calculer une valeur de récompense en fonction du degré de similarité desdites corrélations calculées avec des corrélations cibles correspondant audit attribut cible, et
iii) présenter des informations indicatives d'une ampleur de ladite valeur de récompense audit sujet par ledit dispositif de présentation (6).

**9.** Système d'entraînement à l'activité cérébrale (10) selon la revendication 8, dans lequel
ledit dispositif de détection d'activité cérébrale (20) comporte une pluralité de dispositifs de mesure d'activité cérébrale (10) ; et

ledit moyen de génération de discriminateur (32) comporte un moyen d'extraction pour extraire, par sélection variable à partir de corrélations d'activités cérébrales parmi ladite pluralité de régions prescrites, une expression de contraction commune à des conditions de mesure de ladite pluralité de dispositifs de mesure d'activité cérébrale (10), et des attributs de ladite pluralité de sujets.

10. Système d'entraînement à l'activité cérébrale (10) selon la revendication 8 ou 9, dans lequel
ledit moyen de génération de discriminateur (32) comporte un moyen de régression pour générer ledit discriminateur par régression de réalisation d'une sélection variable supplémentaire sur ladite expression de contraction extraite.

11. Système d'entraînement à l'activité cérébrale (10) selon la revendication 9, dans lequel
ledit moyen d'extraction comporte un moyen d'analyse de corrélation pour calculer une matrice de corrélation d'activités au niveau de ladite pluralité de régions prescrites à partir de signaux détectés par ledit dispositif de détection d'activité cérébrale, exécuter une analyse de corrélation canonique régularisée entre des attributs desdits sujets et des éléments non diagonaux de ladite matrice de corrélation et ainsi extraire ladite expression contractée.

12. Système d'entraînement à l'activité cérébrale (10) selon la revendication 10, dans lequel
ledit moyen de régression comporte un moyen d'analyse de régression pour générer un discriminateur par régression logistique clairsemée sur un résultat de ladite analyse de corrélation canonique régularisée et les attributs desdits sujets.

13. Système d'entraînement à l'activité cérébrale (10) selon la revendication 9, dans lequel
ladite pluralité de dispositifs de mesure d'activité cérébrale (10) sont des dispositifs pour mesurer séquentiellement dans le temps des activités cérébrales par imagerie cérébrale fonctionnelle installés à une pluralité d'emplacements différents, respectivement.

14. Système d'entraînement à l'activité cérébrale (10) selon la revendication 8, dans lequel
ledit processus discriminant est une discrimination d'une étiquette de maladie indiquant si ledit premier sujet (2) est sain ou un patient à trouble neurologique/mental.

15. Système d'entraînement à l'activité cérébrale (10) selon la revendication 9, dans lequel
les attributs desdits sujets comportent une étiquette de maladie indiquant si ledit sujet est sain ou un patient à trouble neurologique/mental, une étiquette indiquant une nature individuelle dudit sujet, et des informations caractérisant la mesure par ledit dispositif de détection d'activité cérébrale ; et
ledit processus discriminant est une discrimination d'une étiquette de maladie indiquant si ledit premier sujet (2) est sain ou un patient à trouble neurologique/mental.

16. Système d'entraînement à l'activité cérébrale (10) selon la revendication 11, dans lequel
ladite analyse de corrélation canonique régularisée est une analyse de corrélation canonique avec une régularisation L1.

17. Système d'entraînement à l'activité cérébrale (10) selon la revendication 9, dans lequel
ledit dispositif de mesure d'activité cérébrale prend une image de résonance magnétique de connectivité fonctionnelle d'état de repos.

# FIG. 1

# FIG. 2

<u>32</u>

# FIG. 3

FIG. 4

FIG. 5

CORRELATION MATRIX
(114 HEALTHY INDIVIDUALS/74 PATIENTS)

FEATURE
EXTRACTION
BY SCCA

DISCRIMINATOR
BY SLR

# FIG. 6

S100
START (GENERATION OF DISCRIMINATOR)

S102
READ DATA OF rs-fcMRI

S104
FEATURE EXTRACTION BY SPARSE CANONICAL CORRELATION ANALYSIS

S106
DISCRIMINANT ANALYSIS BY SPARSE LOGISTIC REGRESSION

S108
GENERATE DISCRIMINATOR

END

SCCA+SLR CANONICAL VARIABLE    FIG. 7

SLR

LABEL
(EXAMPLE : ASD/NC)

SCCA

SUBJECT ATTRIBUTES
(EXAMPLE : ASD/NC)

CANONICAL VARIABLE
TWO DIMENSIONS

CANONICAL ELEMENT
(FEATURE SPACE)

rs-fMRI (4278 CONNECTIONS (NINETY-THREE ROI'S)
->APPROXIMATELY 700 CONNECTIONS ARE SELECTED)

SCCA (MULTI VARIABLES)
+ SLR CANONICAL
VARIABLE

FIG. 8

SLR

LABEL
(EXAMPLE:ASD/NC)

SCCA

SUBJECT ATTRIBUTES
(EXAMPLE: ASD/NC, AGE,
SEX, MEASUREMENT SITE,
INTENSITY OF MAGNETIC
FIELD FOR MEASUREMENT,
EYES OPEN/CLOSED)

CANONICAL VARIABLE
ELEVEN DIMENSIONS

CANONICAL ELEMENT
(FEATURE SPACE)

rs-fMRI (4278 CONNECTIONS (NINETY-THREE ROI' S)
->APPROXIMATELY 700 CONNECTIONS ARE SELECTED)

SCCA (MULTI VARIABLES)
+ SLR CANONICAL
VARIABLE

FIG. 9

SLR

LABEL
(EXAMPLE:ASD/NC)

SCCA

SUBJECT ATTRIBUTES
(EXAMPLE: ASD/NC, AGE,
SEX, MEASUREMENT SITE,
INTENSITY OF MAGNETIC
FIELD FOR MEASUREMENT,
EYES OPEN/CLOSED)

21 CONNECTIONS

CANONICAL ELEMENT
(FEATURE SPACE)

rs-fMRI (4278 CONNECTIONS (NINETY-THREE ROI'S)->APPROXIMATELY
700 CONNECTIONS ARE SELECTED) (FEATURE SPACE)

EP 2 992 823 B1

# FIG. 10

SITE ONE

SITE TWO

SITE THREE

GROUP OF 114 HEALTHY INDIVIDUALS

GROUP OF SEVENTY-FOUR PATIENTS

TIME SEQUENTIAL DATA OF rsfMRI

93

93

EXTRACT AVERAGE WAVEFORM IN EACH REGION OF INTEREST

CALCULATE CORRELATION MATRIX AMONG NINETY-THREE REGIONS

DATA JUST SHY OF 200 INDIVIDUALS

(FOR EACH SUBJECT)

OVER 4000 CONNECTIONS

# FIG. 11

FIG. 12

| | OVERALL | SITE ONE | SITE TWO | SITE THREE | SITE FOUR |
|---|---|---|---|---|---|
| DISCRIMINANT PERFORMANCE | 79.3% | 83.8% | 72.0% | 84.9% | 66.2% |
| SENSITIVITY | 0.730 | 0.771 | 0.692 | | 0.529 |
| SPECIFICITY | 0.833 | 0.889 | 0.750 | 0.849 | 0.794 |
| POSITIVE LIKELIHOOD RATIO LR+ | 4.38 | 6.94 | 2.77 | | 2.57 |
| NEGATIVE LIKELIHOOD RATIO LR- | 0.324 | 0.257 | 0.410 | | 0.593 |
| DOR | 13.5 | 27.0 | 6.75 | | 4.34 |

EP 2 992 823 B1

# FIG. 13

(1) EPI IMAGING

(6) FEEDBACK

(2) RE-CONFIGURATION OF IMAGES (REAL-TIME OUTPUT)

TIME SEQUENTIAL DATA

(3) EXTRACT AVERAGE WAVEFORM

(4) CALCULATE CONNECTIVITY STRENGTH

$$Score \sim \left[ \frac{1}{1+\exp\left(-\beta\,Cw\right)} \right]$$

(5) SCORING (HIGHER SCORE = CLOSER TO HEALTHY INDIVIDUAL)

# FIG. 14

S200
COLLECT DATA WITHOUT SCRUBBING

S202
NORMALIZE DATA
(MEAN ZERO, VARIANCE ONE)

S204
LINEAR SUM OF BIOMARKER
(WITH BIAS TERM)

S206
1ST SESSION?                                    N

Y

S208
DERIVE BIAS TERM FOR
CALIBRATION

S210
CORRECTION (CONSIDERING DIF-
FERENCE BETWEEN RESTING
STATE AND TASK PERFORMANCE)

S212
CALCULATE SCORE SC
FROM BIOMARKER OUTPUT

S214
Y                 $SC \geqq 50$ ?

N
S216
CONVERT TO DEVIATION

S218
CALCULATE/OUTPUT
FEEDBACK SCORE

FIG. 15

```
┌──────────────────────────────────────────────┐
│ PROCESS FOR DERIVING BIAS TERM                 │
│ FOR CALIBRATION                                │
└──────────────────────────────────────────────┘
```

┌─────────────────────────────┐          ┌──────────────────────────────┐
│ COLLECT DATA                 │  S302    │ COLLECT RESTING STATE DATA    │  S312
│ (WITHOUT SCRUBBING)X10       │          │ WITH SCRUBBING                │
└─────────────────────────────┘          └──────────────────────────────┘

┌─────────────────────────────┐          ┌──────────────────────────────┐
│ NORMALIZE DATA               │  S304    │ NORMALIZE DATA                │  S314
│ (MEAN ZERO, VARIANCE ONE)    │          │ (MEAN ZERO, VARIANCE ONE)     │
└─────────────────────────────┘          └──────────────────────────────┘

┌─────────────────────────────┐          ┌──────────────────────────────┐
│ LINEAR SUM OF BIOMARKER      │  S306    │ LINEAR SUM OF BIOMARKER       │  S316
└─────────────────────────────┘          └──────────────────────────────┘

┌─────────────────────────────┐          ┌──────────────────────────────┐
│ STORE SESSION LWS            │  S308    │ STORE RESTING STATE LWS       │  S318
│ (Session LWS)                │          │ (Rest LWS)                    │
└─────────────────────────────┘          └──────────────────────────────┘

┌────────────────────────────────────────────────┐
│ CALCULATE BIAS VALUE                   S320      │
│ FOR CALIBRATION                                  │
│ Bias = (Rest LWS - Session LWS)                  │
└────────────────────────────────────────────────┘

## FIG. 16

```
                                    ┌─S412
┌──────────────────────────────────┐
│ COLLECT RESTING STATE DATA        │
│ WITH SCRUBBING                    │
└──────────────────────────────────┘
                 │
                 ▼              ┌─S414
┌──────────────────────────────────┐
│ NORMALIZE DATA                    │
│ (MEAN ZERO, VARIANCE ONE)         │
└──────────────────────────────────┘
                 │
                 ▼              ┌─S416
┌──────────────────────────────────┐
│ LINEAR SUM OF BIOMARKER           │
└──────────────────────────────────┘
                 │
                 ▼              ┌─S418
┌──────────────────────────────────┐
│ CALCULATE SCORE                   │
└──────────────────────────────────┘
```

# FIG. 17

MEASUREMENT OF RESTING STATE ACTIVITIES

| PRE-TRAINING RESTING STATE | | | | POST TRAINING RESTING STATE | AFTER TWO MONTHS, RESTING STATE |

NEUROFEEDBACK TRAINING

| TRAINING | TRAINING | TRAINING | TRAINING |

DAY ONE   DAY TWO   DAY THREE   DAY FOUR   ● ● ●   DAY FIVE

EP 2 992 823 B1

FIG. 18

LEFT LATERAL VIEW

LEFT MEDIAL VIEW

FIG. 19

FIG. 20

FIRST TRAINING BLOCK ON DAY 1 → LAST TRAINING BLOCK ON DAY 4

RESTING STATE

MOTION IMAGINATION PERIOD

$r = -0.31$

$r = 0.62$

M1
LP

FILTERED BOLD SIGNAL LEVEL

TIME (second)

## FIG. 21

## FIG. 22

NETWORKS REGIONS

# FIG. 23

× : INDIVIDUAL rs-fcMRI
◯ : AVERAGE OF THE DAY

# EP 2 992 823 B1

## REFERENCES CITED IN THE DESCRIPTION

### Patent documents cited in the description

- WO 2006132313 A

- JP 2011000184 A

### Non-patent literature cited in the description

- **NIKOLAUS WEISKOPF.** Real-time fMRI and its application to neurofeedback. *NeuroImage,* 2012, vol. 62, 682-692
- **DECHARMS RC ; MAEDA F ; GLOVER GH et al.** Control over brain activation and pain learned by using real-time functional MRI. *Proc Natl Acad Sci USA,* 2005, vol. 102 (51), 18626-18631
- **RAICHLE ME ; MACLEOD AM ; SNYDER AZ et al.** A default mode of brain function. *Proc Natl Acad Sci USA,* 2001, vol. 98 (2), 676-682
- **KAZUHISA SHIBATA ; TAKEO WATANABE ; YUKA SASAKI ; MITSUO KAWATO.** Perceptual Learning Incepted by Decoded fMRI Neurofeedback Without Stimulus Presentation. *SCIENCE,* 09 December 2011, vol. 334
- **T. WATANABE ; J. E. NANEZ SR ; S. KOYAMA ; I. MUKAI ; J. LIEDERMAN ; Y. SASAKI.** Greater plasticity in lower-level than higher-level visual motion processing in a passive perceptual learning task. *Nature Neuroscience,* 2002, vol. 5, 1003-1009
- **KAMITANI Y ; TONG F.** Decoding the visual and subjective contents of the human brain. *Nat Neurosci.,* 2005, vol. 8, 679-85

- **MICHELLE HAMPSON.** Real-time fMRI Biofeedback Targeting the Orbitofrontal Cortex for Contamination Anxiety. *Journal of visualized experiments,* 20 January 2012
- Sparse estimation automatically selects voxels relevant for the decoding of fMRI activity patterns. **YAMASHITA, O.** Neuroimage. Elsevier, 01 October 2008, vol. 42, 1414-1429
- Unsupervised analysis of fMRI data using kernel canonical correlation. **HARDOON, D. R. et al.** Neuroimage. Elsevier, 14 September 2007, vol. 37, 1250-1259
- **WITTEN DM ; TIBSHIRANI R ; T HASTIE.** A penalized matrix decomposition, with applications to sparse principal components and canonical correlation analysis. *Biostatistics,* 2009, vol. 10 (3), 515-534
- **OKITO YAMASHITA ; MASAAKI SATO ; TAKU YOSHIOKA ; FRANK TONG ; YUKIYASU KAMITANI.** Sparse Estimation automatically selects voxels relevant for the decoding of fMRI activity patterns. *NeuroImage,* 2008, vol. 42 (4), 1414-1429